(19) Europäisches Patentamt
European Patent Office
Office européen des brevets

(11) Veröffentlichungsnummer: **0 298 350 A2**

(12) # EUROPÄISCHE PATENTANMELDUNG

(21) Anmeldenummer: 88110325.3

(22) Anmeldetag: 29.06.88

(51) Int. Cl.4: C07D 211/56 , C07D 491/056 , C07D 491/048 , A61K 31/445 , //(C07D491/056,339:00,221:00), (C07D491/04,307:00,221:00)

(30) Priorität: 10.07.87 DE 3722788

(43) Veröffentlichungstag der Anmeldung: 11.01.89 Patentblatt 89/02

(84) Benannte Vertragsstaaten: AT BE CH DE ES FR GB GR IT LI NL SE

(71) Anmelder: BAYER AG
Konzernverwaltung RP Patentabteilung
D-5090 Leverkusen 1 Bayerwerk(DE)

(72) Erfinder: Schüller, Matthias, Dr.
Gellertweg 20
D-5600 Wuppertal(DE)
Erfinder: Böshagen, Horst, Dr.
Wiesenstrasse 4
D-5657 Haan(DE)
Erfinder: Heiker, Fred-Robert, Dr.
Pahlkestrasse 15
D-5600 Wuppertal 1(DE)

(54) **Chirale 6-Hydroxymethylenverzweigte 3-Amino-4,5-dihydroxypiperidine, Zwischenprodukte zu ihrer Herstellung, Verfahren zu ihrer Herstellung und ihre Verwendung.**

(57) Die Erfindung betrifft chirale 6-Hydroxymethylenverzweigte 3-Amino-4,5-dihydroxypiperidine der Formel

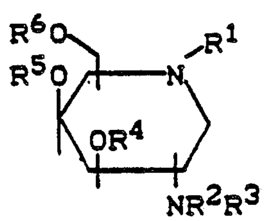

(I)

in welcher $R^1$, $R^2$, $R^3$, $R^4$, $R^5$ und $R^6$ die in der Beschreibung angegebene Bedeutung haben, Zwischenprodukte zu ihrer Herstellung, Verfahren zu ihrer Herstellung und ihre Verwendung in Arzneimitteln, insbesondere zur Beeinflussung des Lipid- und Kohlenhydratstoffwechsels.

EP 0 298 350 A2

# Chirale 6-Hydroxymethylenverzweigte 3-Amino-4,5-dihydroxypiperidine, Zwischenprodukte zu ihrer Herstellung, Verfahren zu ihrer Herstellung und ihre Verwendung

Die Erfindung betrifft chirale 6-Hydroxymethylenverzweigte 3-Amino-4,5-dihydroxypiperidine, Zwischenprodukte zu ihrer Herstellung, Verfahren zu ihrer Herstellung und ihre Verwendung in Arzneimitteln, insbesondere zur Beeinflussung des Lipid- und Kohlenhydratstoffwechsels.

Es ist bekannt, daß 1-Desoxynojirimycin eine Hemmwirkung auf Glukosidasen hat [vgl. G. Truscheit, W. Frommer, B. Junge, L. Müller, D. Schmidt, W. Wingender, Angew. Chem. 93, 738 (1981)].

Die vorliegende Erfindung betrifft chirale 6-Hydroxymethylenverzweigte 3-Amino-4,5-dihydroxypiperidine der allgemeinen Formel (I)

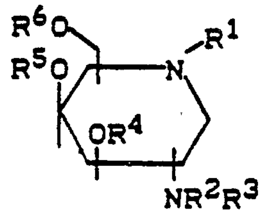

(I)

in welcher

$R^1$ - für Wasserstoff steht, oder

- für geradkettiges oder verzweigtes Alkyl mit bis zu 8 Kohlenstoffatomen steht, das ein- oder mehrfach gleich oder verschieden durch Halogen substituiert sein kann, oder
- für geradkettiges oder verzweigtes Alkenyl mit bis zu 4 Kohlenstoffatomen steht, oder
- für Aralkyl mit 7 bis 14 Kohlenstoffatomen steht, das im Aromaten ein- oder mehrfach gleich oder verschieden durch Halogen oder Alkoxy mit bis zu 4 Kohlenstoffatomen substituiert sein kann, oder
- für eine Gruppe der Formel

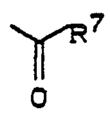

steht,

worin

$R^7$ - geradkettiges oder verzweigtes Alkyl oder Alkenyl mit bis zu 8 Kohlenstoffatomen bedeutet, oder

- geradkettiges oder verzweigtes Alkoxy mit bis zu 8 Kohlenstoffatomen bedeutet, oder
- Aralkoxy mit bis zu 10 Kohlenstoffatomen bedeutet,

$R^2$, $R^3$ - gleich oder verschieden sind und

- für Wasserstoff stehen, oder
- für geradkettiges oder verzweigtes Alkyl mit bis zu 8 Kohlenstoffatomen stehen, die ein- oder mehrfach gleich oder verschieden durch Halogen substituiert sein können, oder
- für geradkettiges oder verzweigtes Alkenyl mit bis zu 6 Kohlenstoffatomen stehen, oder
- für Aralkyl mit 7 bis 14 Kohlenstoffatomen steht, das im Aromaten ein- oder mehrfach gleich oder verschieden durch Halogen oder durch Alkoxy mit bis zu 4 Kohlenstoffatomen substituiert sein können, oder
- für eine Gruppe der Formel

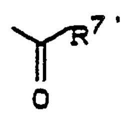

stehen,

worin

$R^{7'}$ - die gleiche Bedeutung hat wie $R^7$ und mit diesem gleich oder verschieden sein kann,

$R^4$ - für Wasserstoff steht, oder

- für geradkettiges oder verzweigtes Alkyl mit bis zu 4 Kohlenstoffatomen steht, das ein- oder mehrfach gleich oder verschieden durch Halogen substituiert sein kann, oder
- für Alkenyl mit bis zu 4 Kohlenstoffatomen steht, oder
- für Aralkyl mit 7 bis 14 Kohlenstoffatomen steht, das im Aromaten ein- oder mehrfach gleich oder verschieden durch Halogen oder durch Alkoxy mit bis zu 4 Kohlenstoffatomen substituiert sein kann, oder
- für eine Gruppe der Formel

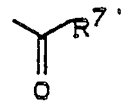 steht,

wobei
$R^{7'}$ - die oben angegebene Bedeutung hat und
$R^5$, $R^6$ - gleich oder verschieden sind und
- für Wasserstoff stehen, oder
- für geradkettiges oder verzweigtes Alkyl oder Alkenyl mit bis zu 4 Kohlenstoffatomen stehen, oder
- für Aralkyl mit 7 bis 14 Kohlenstoffatomen steht, das im Aromaten ein- oder mehrfach gleich oder verschieden durch Halogen oder Alkoxy mit bis zu 4 Kohlenstoffatomen substituiert sein können, oder
- für eine Gruppe der Formel

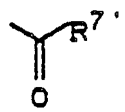 stehen,

wobei
$R^{7'}$ - die oben angegebene Bedeutung hat, oder
$R^5$, $R^6$ - gemeinsam eine Methylen-, Ethylen-, Propyliden-, Isopropyliden-, Cyclohexyliden- oder Benzylidengruppe bilden
und deren physiologisch unbedenkliche Salze.

Bevorzugt sind Verbindungen der allgemeinen Formel (I), in welcher
$R^1$ - für Wasserstoff steht, oder
- für Alkyl mit bis zu 6 Kohlenstoffatomen steht, das ein- oder mehrfach gleich oder verschieden durch Fluor oder Chlor substituiert sein kann, oder
- für Allyl steht, oder
- für Benzyl steht, das im Phenylring ein- oder mehrfach gleich oder verschieden durch Fluor, Chlor, Brom oder Methoxy substiutiert sein kann, oder
- für eine Gruppe der Formel

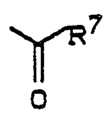 steht,

worin
$R^7$ - geradkettiges oder verzweigtes Alkyl oder Alkenyl mit bis zu 4 Kohlenstoffatomen bedeutet, oder
- geradkettiges oder verzweigtes Alkoxy mit bis zu 4 Kohlenstoffatomen bedeutet, oder
- Benzyloxy bedeutet,
$R^2$ - für Wasserstoff steht,
$R^3$ - für Wasserstoff steht, oder
- für Alkyl mit bis zu 6 Kohlenstoffatomen steht, das ein- oder mehrfach gleich oder verschieden durch Fluor oder Chlor substituiert sein kann,
- für Allyl steht, oder
- für Benzyl steht, das im Phenylring ein- oder mehrfach gleich oder verschieden durch Fluor, Chlor, Brom oder Methoxy substituiert sein kann, oder

- für eine Gruppe der Formel

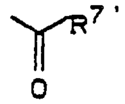

steht,

wobei
$R^{7'}$ - die gleiche Bedeutung hat wie $R^7$ und mit diesem gleich oder verschieden sein kann,
$R^4$ - für Wasserstoff, Methyl, Ethyl oder Allyl steht, oder
- für Benzyl steht, das im Phenylrest ein- oder mehrfach gleich oder verschieden durch Fluor, Chlor, Brom oder Methoxy substituiert sein kann, oder
- für eine Gruppe der Formel

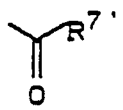

steht,

worin
$R^{7'}$ - die oben angegebene Bedeutung hat,
und
$R^5$, $R^6$ - gleich oder verschieden sind und
- für Wasserstoff, Methyl oder Ethyl stehen, oder
- für Benzyl stehen, das im Phenylrest ein- oder mehrfach, gleich oder verschieden durch Fluor, Chlor oder Methoxy substituiert sein kann, oder
- für eine Gruppe der Formel

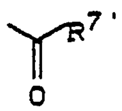

stehen,

worin
$R^{7'}$ - die oben angegebene Bedeutung hat,
oder
$R^5$, $R^6$ - gemeinsam eine Methylen-, Isopropyliden- oder Cyclohexylidengruppe bilden
und deren physiologisch unbedenkliche Salze.

Besonders bevorzugt sind Verbindungen der allgemeinen Formel (I) genannt,
in welcher
$R^1$ - für Wasserstoff steht, oder
- für Alkyl mit bis zu 4 Kohlenstoffatomen steht, oder
- für Benzyl, 4-Chlorbenzyl, 4-Fluorbenzyl oder 4-Methoxybenzyl steht, oder
- für Acetyl, Methoxycarbonyl, Ethoxycarbonyl oder Benzyloxycarbonyl steht,
$R^2$ - für Wasserstoff steht,
$R^3$ - für Wasserstoff steht, oder
- für Alkyl mit bis zu 4 Kohlenstoffatomen steht, oder
- für Benzyl, 4-Chlorbenzyl, 4-Fluorbenzyl oder 4-Methoxybenzyl steht, oder
- für Acetyl steht,
$R^4$ - für Wasserstoff steht, oder
- für Benzyl oder 4-Methoxybenzyl steht, oder
- für Acetyl steht,
und
$R^5$, $R^6$ - gleich oder verschieden sind und
- für Wasserstoff stehen, oder
- für Benzyl oder 4-Methoxybenzyl stehen, oder

- für Acetyl stehen,
oder
$R^5$, $R^6$ - gemeinsam eine Isopropylidengruppe bilden
und deren physiologisch unbedenkliche Salze.

Physiologisch unbedenkliche Salze sind im allgemeinen Salze der erfindungsgemäßen Verbindungen mit anorganischen oder organischen Säuren. Hierzu gehören bevorzugt Hydrochloride, Hydrobromide, Sulfate, Hydrogensulfate, Hydrogenphosphate, Hydrogencarbonate, Carbonate, Phosphate, Acetate, Propionate, Maleate, Citrate, Fumarate, Oxalate, Lacetate, Succinate, Tartrate oder Benzoate.

Die erfindungsgemäßen Substanzen der allgemeinen Formel (I), sowie deren physiologish unbedenkliche Salze
erhält man, indem man
Azidoverbindungen der allgemeinen Formel (II)

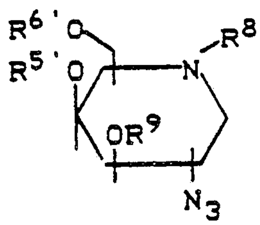

(II)

in welcher
$R^{5'}$ und $R^{6'}$ gemeinsam eine Isopropylidengruppe bilden,
$R^8$ - für eine Aminoschutzgruppe, bevorzugt für Benzyl steht,
und
$R^9$ - für eine Hydroxylschutzgruppe, bevorzugt für Benzyl, 4-Methoxybenzyl oder Allyl steht,
in inerten Lösemitteln, in Anwesenheit eines Reduktionsmittels, gegebenenfalls in Anwesenheit eines Katalysators reduziert,
gegebenenfalls Schutzgruppen abspaltet, oder neue Schutzgruppen einführt,
und gegebenenfalls anschließend mit Säure die entsprechenden Salze herstellt.

Die Herstellung der erfindungsgemäßen Verbindungen kann durch folgendes Formelschema beispielhaft erläutert werden:

<u>Schema 1</u>

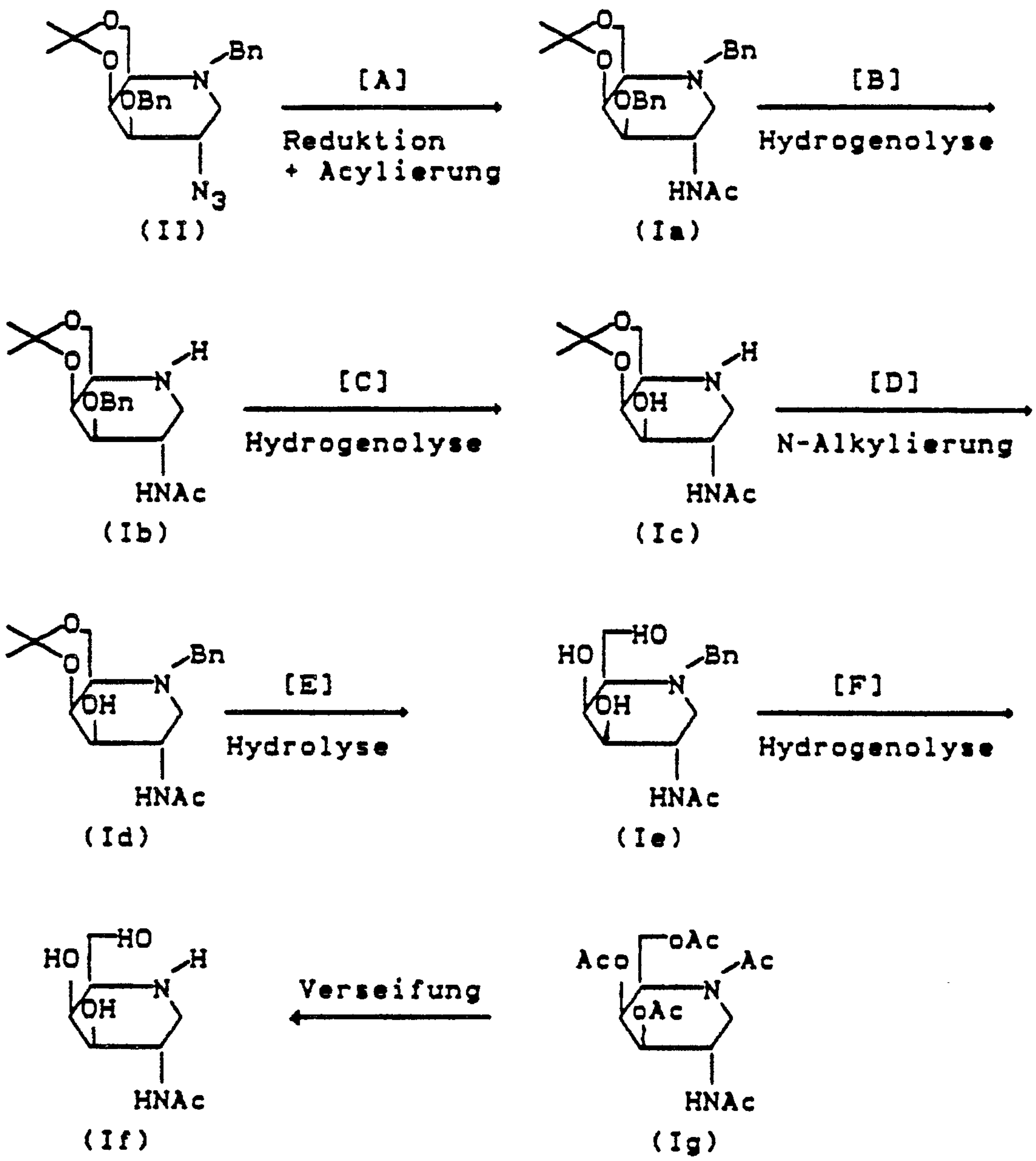


Bn = Benzyl = $CH_2-C_6H_5$
Ac = Acetyl = $COCH_3$

Die Reduktion wird im allgemeinen nach den Methoden durchgeführt, wie sie für die Reduktion von Aziden zu Aminogruppen üblich ist.

Hierzu gehören die Reduktion mit Wasserstoff als Reduktionsmittel, in Anwesenheit eines Katalysators wie Raney-Nickel, Palladium, Platin (gegebenenfalls auf Tierkohle), gegebenenfalls in Anwesenheit von Säuren wie Sulfonsäuren (z.B. Methansulfonsäure, Ethansulfonsäure, Benzolsulfonsäure oder Toluolsulfonsäure), oder Mineralsäuren (z.B. Chlorwasserstoffsäure, Bromwasserstoffsäure oder Schwefelsäure), oder Carbonsäuren (z.B. Essigsäure, Trifluoressigsäure, Trichloresigsäure) in inerten Lösemitteln wie Wasser, Alkohlen, z.B. Methanol, Ethanol, Propanol oder Isopropanol, oder Dimethylformamid, oder Gemische der genannten Lösemittel, oder die Reduktion mit Hydriden als Reduktionsmittel wie beispielsweise Natriumborhydrid, Lithiumaluminiumhydrid oder Natriumcyanoborhydrid, gegebenenfalls in Anwesenheit von Nickelsalzen, in inerten Lösemitteln wie Wasser, oder Alkohlen wie Methanol, Ethanol, Propanol oder Isopropanol,

oder Dimethylformamid, oder Gemische der genannten Lösemittel, sowie die Reduktion mit Schwefelwasserstoff in Pyridin oder Triphenylphosphinoxid in ammoniakalischen Lösungen, niederen Alkoholen, vorzugsweise Methaol.

Weitere erfindungsgemäß anwendbare Reduktionsverfahren für Azide werden in Houben-Weyl's "Methoden oder organischen Chemie" X/3, 822; XI/1, 539 beschrieben.

Die Abspaltung von Schutzgruppen bzw. Neueinführung neuer Schutzgruppen erfolgt im allgemeinen nach üblichen Methoden wie sie z.B. in Hauben-Weyl's "Methoden der organischen Chemie" XV/1 und 1 sowie Baker und Ollis, Comprehensive Organic Chemistry 5 714 ff. beschrieben sind.

Beispielsweise kann das Abspalten und Austauschen von Schutzgruppen wie es beispielhaft in Schema 1 dargestellt ist, folgendermaßen durchgeführt werden:

Nach der erfindungsgemäßen Reduktion der Azidoverbindung (IIa) in Schritt [A] mit nachfolgender Acetylierung in situ mit Essigsäureanhydrid und Pyridin zur erfindungsgemäßen Verbindung (Ia) kann die Benzylgruppe am Stickstoff beispielsweise durch Reduktion mit Wasserstoff in Anwesenheit von Palladium auf Tierkohle bei 0.5 mega-Pascal (MPa) Wasserstoffdruck in Methanol entfernt werden (Schritt B). In Schritt [C] wird dann durch Hydrieren bei 0.5 mega-Pascal (MPa) Wasserstoffdruck in Methanol, dem eine äquimolare Menge Essigsäure zugesetzt wurde und in Gegenwart von Palladium auf Tierkohle, die O-Benzylgruppe abgespalten und Verbindung (Ic) erhalten. Diese Verbindung wird dann in Schritt [D] in N,N-Dimethylformamid mit Benzylbromid und Kaliumcarbonat als Säurefänger bei 0° C erneut am Stickstoff zu Verbindung (Id) benzyliert. Die Behandlung von Verbindung (Id) mit einer Mischung aus Essigsäure, Chlorwasserstoffsäure und Wasser bei erhöhter Temperatur führt in Schritt [E] zu der Verbindung (Ie), aus der durch Hydrieren in einer Mischung aus Dioxan und Wasser in Gegenwart von Palladium auf Tierkohle in Schritt [F] die Verbindung (If) hervorgeht. Verbindung (If) kann direkt auch aus Verbindung (XIV) in Schema (II) hergestellt werden. Dazu wird (XIV) in einer Mischung aus Dioxan und 1-N-Chlorwasserstoffsäure in Volumenverhältnis 1:1 in Gegenwart von Palladium auf Tierkohle bei 1 mega-Pascal (MPa) Wasserstoffdruck hydriert. Dann wird mit 1-N-Natriumhydroxid-Lösung neutralisiert, eingeengt, und der Rückstand mit Pyridin und Essigsäureanhydrid behandelt. Die so erhaltene peracetylierte Verbindung (Ig) liefert nach Rühren mit Natriummethanolat in Methanol die Verbindung (If).

Die als Ausgangsstoffe eingesetzten Azidoverbindungen der allgemeinen Formel (II) sind neu. Sie können jedoch nach im Prinzip bekannten Methoden gemäß folgendem Schema hergestellt werden:

Schema 2

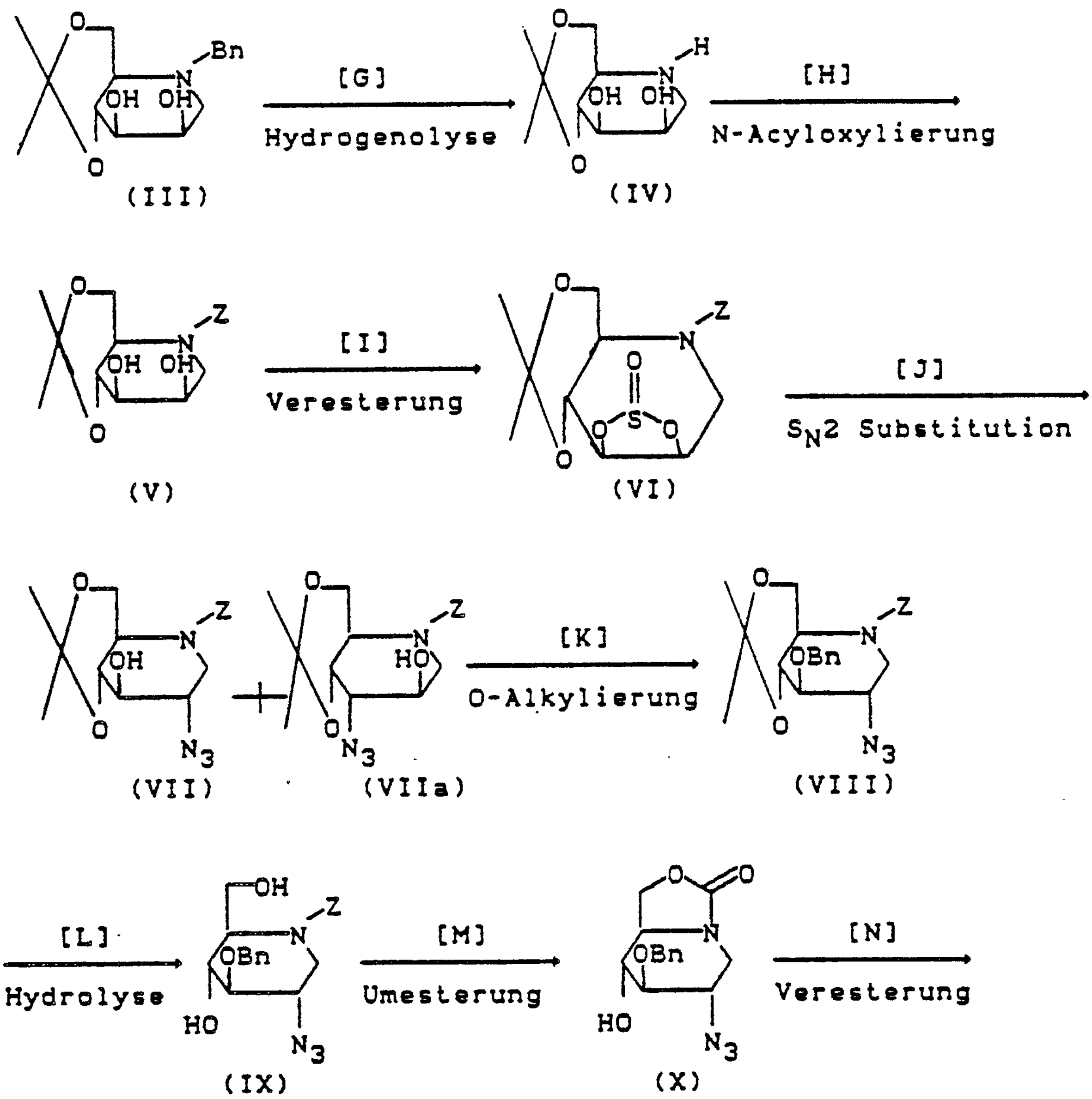


(III) (IV) (V) (VI) (VII) + (VIIa) (VIII) (IX) (X)

Schema 2

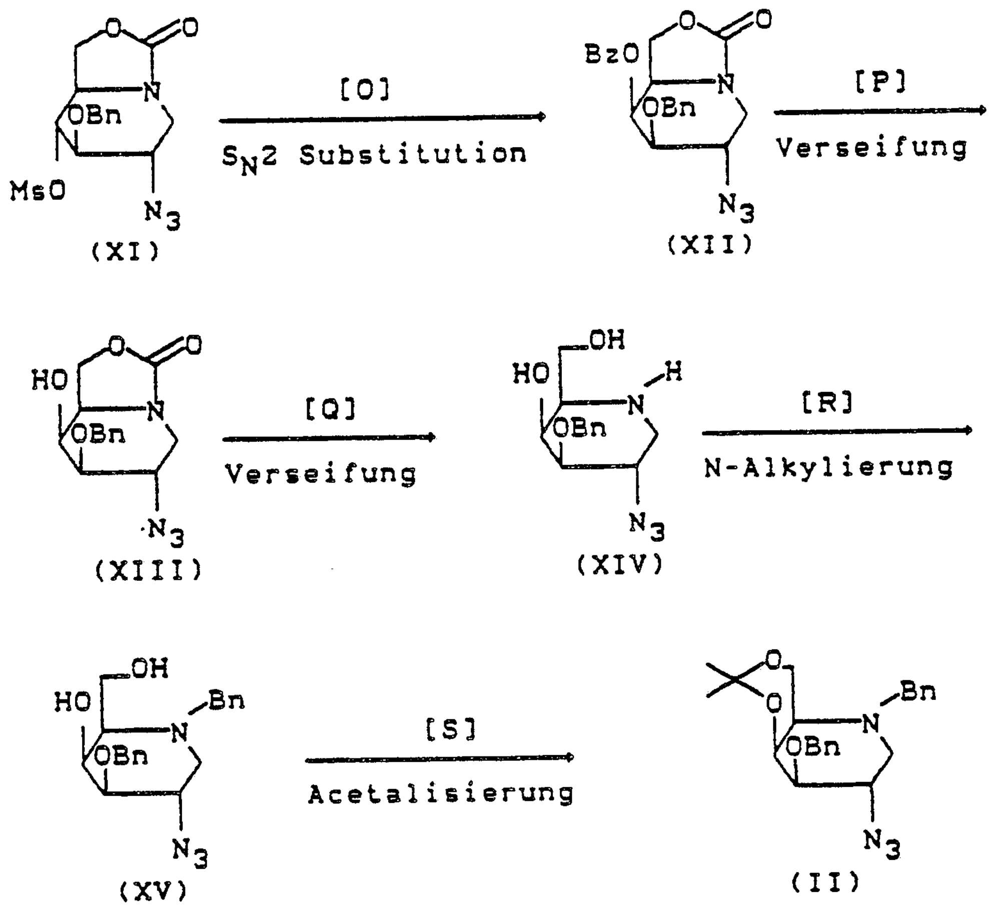


Bn = Benzyl = $-CH_2-C_6H_5$
Bz = Benzoyl = $-CO-C_6H_5$
Z = Benzyloxycarbonyl = $-CO-O-CH_2-C_6H_5$

Danach wird das D-manno-konfigurierte Derivat (III) in Methanol [Schritt G] am Platin/Kohle-Kontakt hydriert und das Produkt (IV) in Schritt [H] mit Chlorameisensäurebenzylester und Kaliumcarbonat in N,N-Dimethylformamid bei Eiskühlung zu (V) umgesetzt, das anschließend in Schritt [I] mit Thionylchlorid in Ethylacetat bei Eiskühlung zu (VI) umgesetzt wird. Das Sulfit (VI) wird in Schritt [J] in N,N-Dimethylformamid mit Lithiumazid in (VII) überführt und diese Verbindung anschließend in Schritt [K] mit Benzylbromid und Kaliumhydroxid in Tetrahydrofuran bei etwa 40°C zu der Verbindung (VIII) benzyliert. Die Abspaltung der Isopropylidenschutzgruppe in Essigsäure/Wasser/Dichloromethan/Salzsäure-Gemischen führt in Schritt [L] zu der Verbindung (IX), die in Schritt [M] in N,N-Dimethylformamid mit Kaliumcarbonat zu Verbindung (X) cyclisiert wird. Die Umsetzung von Verbindung (X) mit Methansulfonsäurechlorid führt in Schritt [N] bei 0°C in Aceton mit Triethylamin als Säurefänger zum Methansulfonat (XI). Wird dieses in N,N-Dimethylformamid bei erhöhter Temperatur, vorzugsweise 100°C, mit Lithiumbenzoat umgesetzt, so wird unter Inversion der Konfiguration in Position 4 in Schritt [O] der D-galakto konfigurierte Benzoesäureester (XII) erhalten. Seine Verseifung mit einer Stöchiometrischen Menge 10 N Natriumhydroxid-Lösung führt in Schritt [P] zu der selektiv deblockierten Verbindung (XIII), deren Umsetzung mit Bariumhydroxid in Methanol unter Rückfluß im Schritt [Q] die Azido-Verbindung (XIV) ergibt. Ihre Umsetzung mit Benzylbromid in N,N-Dimethylformamid bei Gegenwart von Kaliumcarbonat als Säurefänger liefert in Schritt [R] bei niedriger Temperatur, vorzugsweise 0 - 10°C, die Verbindung (XV). Aus Verbindung (XV) wird in Schritt [S] durch Umsetzung mit 2,2 -Dimethoxypropan in N,N-Dimethylformamid bei pH 2,5 und 40°C die Verbindung (II) erhalten.

Das als Ausgangsstoff eingesetzte manno-konfigurierte Derivat (III) ist neu und kann gemäß Schema 3 nach im Prinzip bekannten Methoden hergestellt werden:

**Schema 3**

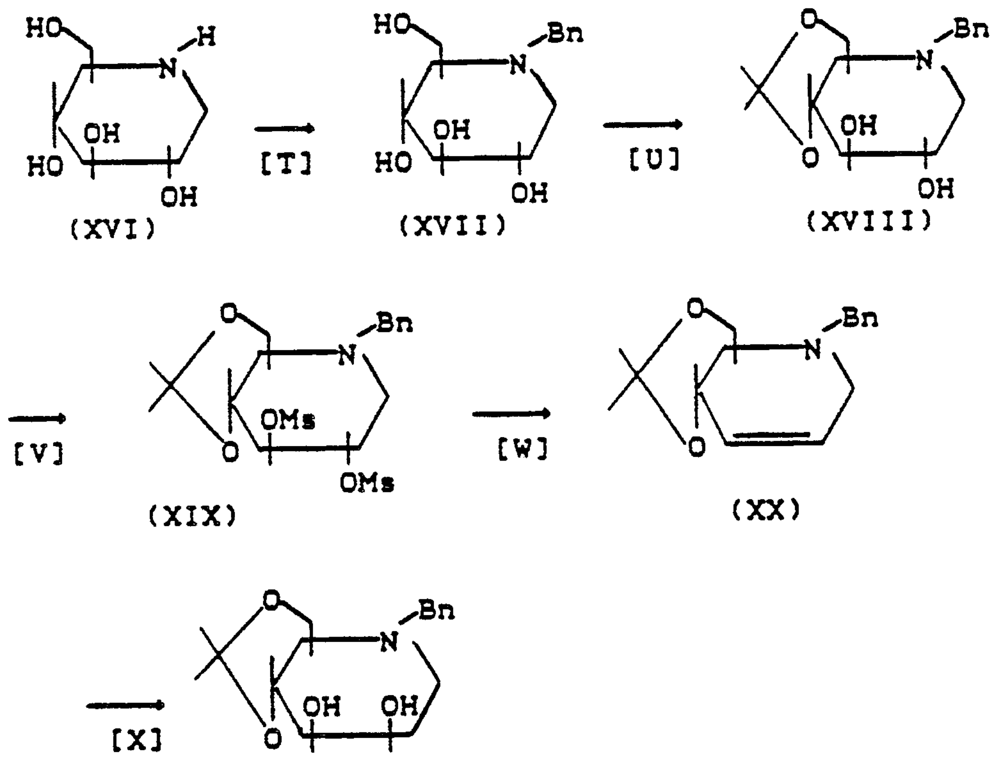


(XVI) (XVII) (XVIII) (XIX) (XX) (III)

Danach wird im Schritt [T] Desoxnojirimycin (XVI) (bekannt aus EP-OS 947) mit Benzylbromid und Kaliumcarbonat in Dimethylformamid bei Eiskühlung zu (XVII) benzyliert. Im Schritt [U] wird die Benzylverbindung (XVII) nach Abtrennung des Kaliumcarbonats mit Säuren (pH = 2) mit 2-Methoxypropen und/oder 2,2-Dimethoxypropan in Dimethylformamid zu (XVIII) umgesetzt und Verbindung (XVIII) in Schritt [V] mit Methansulfonsäurechlorid in Acetonitril/Triethylamin in das Mesylat (XIX) überführt. Durch Umsetzung von (XIX) mit Natriumiodid/Zink in Dimethylformamid [Schritt W] erhält man das Olefin (XX), das anschließend in Schritt [X] mit Osmiumtetroxid/N-Methylmorpholin-N-oxid zu (III) oxidiert wird.

Die Zwischenverbindungen der Formeln (IV - XIV) sind neu. Eine besonders wichtige Zwischenverbindung für die Herstellung der erfindungsgemäßen Verbindungen der allgemeinen Formel (I) nach dem erfindungsgemäßen Verfahren ist das D-galakto-konfiguriert Carbamat der allgemeinen Formel (XII). Von diesem ausgehend sind die Verbindungen der allgemeinen Formel (I) mit Methoden der Schutzgruppenchemie, wie sie vorzugsweise in der Kohlenhydratchemie Anwendung finden und im Prinzip bekannt sind, zugänglich.

Die D-galakto-konfigurierten Azidoverbindungen der allgemeinen Formel (XII) erhält man, indem man D-gluko-konfigurierten 3-Azidopiperidine der allgemeinen Formel (VIII) ausgeht, und diese in Carbamate (X) überführt, die in der 5-Position selektiv deblockiert sind.

Die Veresterung mit einer Abgangsgruppe wie Tosyl, Mesyl, Trifluormethansulfonyl, vorzugsweise aber mit Mesyl führt zu aktivierten Estern der Formel (XI), die mit geeignetem O-Nukleophilen, wie sie dem Fachmann geläufig sind, unter Inversion reagieren. Geeignete O-Nukleophile sind z.B. Salze aromatischer Carbonsäuren, speziell das Lithiumsalz der Benzoesäure.

Die erfindungsgemäßen Verbindungen haben interessante pharmakologische Wirkungen. Sie hemmen den extra- und intrazellulären Kohlenhydratstoffwechsel, Glykosyltransferasen, sowie Glykosidasen.

So ist z.B. die Verbindung des Beispiels (If) ein potenter Inhibitor der α-N-Acetyl-galaktosaminidase aus Charonia lampas mit einer $IC_{50}$ von 1,4 µM. Sie inhibiert außerdem die Aktivität der UDP-gal NAc: polypeptid-α-N-acetylgalaktosaminyl-transferase und erhöht die Halbwertszeit von Glykoproteinen.

Die Verbindungen der allgemeinen Formel (I) beeinflussen die Glykosylierung von Proteinen und damit die O- und N-Glykoprotein-Biosynthese und verändern z.B. die Zusammensetzung der Schleimstoffe. Sie

nehmen außerdem Einfluß auf den Chitinstoffwechsel bei Insekten, wirken fungizid und/oder bakteriostatisch und können als Immunmodulatoren oder zur Verhinderung der Befruchtung und/oder Nidation des befruchteten Eies eingesetzt werden. Prädiabetes, Diabetes, Obstipation, Karies, Atherosklerose, Adipositas, Hyperlipoproteinamie, chronisch obstruktive Lungenerkrankungen infektiöser wie atopischer Art, Immunmangelzustände und Infektionskrankheiten bakterieller wie viraler Genese (z.B. Hepatitis B, HLV Infektionen, Erkältungserkrankungen) werden durch die Verbindungen der allgemeinen Formel (I) günstig beeinflußt. Die Verbindungen der Formel (I) beeinflussen die Glykosylierung auch von viralen Proteinen und sind deswegen zur Therapie von viralen Erkrankungen geeignet.

Die Verbindungen können ohne Verdünnung, z.B. als Pulver oder in einer Gelatinehülle oder in Kombination mit einem Trägerstoff in einer pharmazeutischen Zusammensetzung appliziert werden. Pharmazeutische Zusammensetzungen können eine größere oder kleinere Menge des Wirkstoffes enthalten, z.B. 0,1% bis 99,5%, in Kombination mit einem pharmazeutisch verträglichen nicht-toxischen, inerten Trägerstoff, wobei der Trägerstoff eine oder mehrere feste, halbfeste oder flüssige Verdünnungsmittel, Füllstofffe und/oder nichttoxische, inerte und pharmazeutisch verträgliche Formulierungshilfsmittel enthalten kann. Solche pharmazeutischen Zubereitungen liegen bevorzugt in Form von Dosierungseinheiten vor, d.h. physikalisch-diskreten, eine bestimmte Menge des Wirkstoffes enthaltenden Einheiten, die einem Bruchteil oder einem Vielfachen der Dosis entsprechen, die zur Herbeiführung der gewünschten Wirkung erforderlich sind. Die Dosiseinheiten können 1,2,3,4 oder mehr Einzeldosen oder 1/2, 1/3 oder 1/4 einer Einzeldosis enthalten. Eine Einzeldosis enthält vorzugsweise eine genügende Menge Wirkstoff, um bei einer Applikation gemäß eines vorher bestimmten Dosierungsschemas einer oder mehrerer Dosierungseinheiten die gewünschte Wirkung zu erzielen, wobei ein Ganzes, ein Halbes, ein Drittel oder ein Viertel der Tagesdosis gewöhnlich zu allen, Haupt- und Nebenmahlzeiten am Tag verabreicht wird.

Andere therapeutische Mittel können auch eingenommen werden. Obgleich die Dosierung und das Dosierungsschema in jedem Fall sorgsam abgewogen werden sollte, unter Anwendung gründlichen fachmännischen Urteils und unter Beachtung des Alters, des Gewichts und des Zustands des Pati enten, der Art und der Schwere der Erkrankung, wird die Dosierung gewöhnlich in einem Bereich zwischen etwa 1 bis etwa 1 x $10^4$ SIE/kg des Körpergewichtes pro Tag liegen. In manchen Fällen wird man dabei eine ausreichende therapeutische Wirkung mit einer geringeren Dosis erreichen, während in anderen Fällen eine größere Dosis erforderlich sein wird.

Orale Applikation kann unter Verwendung fester und flüssiger Dosierungseinheiten durchgeführt werden z.B. als Pulver, Tabletten, Dragees, Kapseln, Granulate, Suspensionen, Lösungen und dergleichen.

Pulver wird durch Zerkleinerung der Substanz zu einer geeigneten Größe und Vermischen mit einem ebenfalls zerkleinerten pharmazeutischen Trägerstoff hergestellt. Obgleich ein eßbares Kohlenhydrat, wie z.B. Stärke, Lactose, Saccharose oder Glukose normalerweise zu diesem Zweck Verwendung findet und auch hier benutzt werden kann, ist es wünschenswert ein Kohlenhydrat, das nicht metabolisiert wird, wie z.B. ein Cellulosederivat, zu benutzen.

Süßmittel, Geschmackzusätze, Konservierungsstoffe, Dispergiermittel und Färbemittel können auch mitverwendet werden.

Die Kapseln können durch Zubereitung der oben beschriebenen Pulvermischungen und durch Füllung bereits gebildeter Gelatinehüllen hergestellt werden. Die Pulvermischungen kann man vor dem Füllvorgang mit Gleitmitteln, wie z.B. Kieselgel, Talkum, Magnesiumstearat, Calciumstearat oder festem Polyethylenglykol versetzen.

Die Mischung kann ebenfalls mit einem Desintegrator oder Lösungsvermittler, wie z.B. Agar-Agar, Calciumcarbonat oder Natriumcarbonat versetzen, um bei Einnahme der Kapsel die Zugänglichkeit des Wirkstoffes zu verbessern.

Die Anfertigung der Tabletten erfolgt z.B. durch Herstellen einer Pulvermischung, grob oder feinkörnig, und Hinzufügen eines Gleitmittels und Desintegrators. Aus dieser Mischung formt man Tabletten. Eine Pulvermischung bereitet man vor durch Mischung der Substanz, welche in geeigneter Weise zerkleinert wurde ,und ergänzt ein Verdünnungsmittel oder eine andere Trägersubstanz wie oben beschrieben. Gegebenenfalls fügt man ein Bindemittel z.B. Carboxymethylcellulose, Alginate, Gelatine oder Polyvinylpyrrolidone, einen Lösungsverzögerer, wie z.B. Paraffin, einen Resorptionsbeschleuniger, wie z.B. ein quarternäres Salz und/oder ein Adorptionsmittel wie z.B. Bentonit, Kaolin oder Dicalciumphosphat hinzu. Die Pulvermischung kann granuliert werden zusammen mit einem Bindemittel, wie z.B. Sirup, Stärkepaste, Akazienschleim oder Lösungen aus Zellulose-oder Polymerenmaterial. Danach preßt man das Produkt durch ein grobes Sieb. Als Alternative hierzu kann man die Pulvermischung durch eine Tablettenmaschine laufen lassen und die sich ergebenden ungleichmäßig geformten Stücke bis auf Korngröße zerkleinern. Damit die entstandenen Körner nicht in den Düsen stecken bleiben, kann man sie mit einem Gleitmittel versetzen, wie z.B. Stearinsäure, Stearatsalz, Talkum oder Mineralöl. Diese gleitfähig gemachte Mischung

wird dann in Tablettenform gepreßt. Die Wirkstoffe können auch mit freifließenden, inerten Trägerstoffen vereinigt werden und direkt in Tablettenform ge bracht werden unter Auslassung der Granulat- und Zerstückelungsschritte. Man kann das Produkt mit einer klaren oder opaken Schutzhülle versehen, z.B. einem Überzug aus Schellack, einem Überzug aus Zucker oder Polymersubstanzen und einer polierten Hülle aus Wachs. Farbstoffe können diesen Überzügen beigefügt werden, damit zwischen den einzelnen Dosierungseinheiten unterschieden werden kann.

Die oral verabreichten Zubereitungsformen, z.B. Lösungen, Sirupe und Elixiere, lassen sich in Dosierungseinheiten herstellen, so daß eine bestimmte Menge Präparat eine bestimmte Menge Wirkstoff enthält. Sirup kann so hergestellt werden, daß der Wirkstoff in einer wäßrigen Lösung, welche geeignete Geschmacksstoffe enthält, gelöst wird. Elixiere werden unter Verwendung nichttoxischer, alkoholischer Trägerstoffe erhalten. Suspensionen kann man durch Dispergieren der Verbindung in einem nichttoxischen Trägerstoff darstellen. Lösungsvermittler und Emulgiermittel, wie z.B. ethoxylierte Isostearylalkohole und Polyoxyethylensorbitester, Konservierungsmittel, geschmacksverbessernde Zusätze, wie z.B. Pfefferminzöl oder Saccharin und dergleichen können auch zugegeben werden.

Dosierungsvorschriften können auf der Kapsel angegeben werden. Überdies kann die Dosierung so abgesichert sein, daß der Wirkstoff verzögert abgegeben wird, z.B. durch Einhalten des Wirkstoffes in Polymersubstanzen, Wachse und dergleichen.

Zusätzlich zu den oben angegebenen pharmazeutischen Zusammensetzungen lassen sich auch diese enthaltende Lebensmittel herstellen, beispielsweise Zucker, Brot, Kartoffelpro dukte, Fruchtsaft, Bier, Schokolade oder andere Konfektartikel und Konserven, wie beispielsweise Marmelade, wobei zu diesen Produkten eine wirksame Menge mindestens eines der erfindungsgemäßen Wirkstoffe gegeben wird.

Die unter Verwendung der erfindungsgemäßen Wirkstoffe hergestellten Nahrungsmittel eignen sich sowohl zur Diät bei Patienten, die an Stoffwechselstörungen leiden, als auch zur Ernährung gesunder Personen im Sinne einer Stoffwechselstörung vorbeugenden Ernährungsweise.

Die erfindungsgemäßen Verbindungen weisen weiterhin die Eigenschaften auf, in Tieren das Verhältnis des Anteils an gewünschtem Fett zum Anteil des gewünschten fettarmen Fleisches zugunsten des mageren Fleisches in hohem Maß zu beeinflussen. Dies ist von besonderer Bedeutung für die Aufzucht und Erhaltung von sonstigen Nutz- und Ziertieren. Die Verwendung der Wirkstoffe kann weiterhin erheblich zur Rationalisierung der Fütterung der Tiere führen, sowohl zeitmäßig, mengenmäßig als auch qualitätsmäßig. Da sie eine gewisse Verzögerung der Verdauung bewirken, wird die Verweildauer der Nährstoffe im Verdauungstrakt verlängert, wodurch eine mit weniger Aufwand verbundene ad libitum Fütterung ermöglicht wird. Weiterhin ergibt sich bei der Verwendung der erfindungsgemäßen Stoffe in vielen Fällen eine erhebliche Einsparung von wertvollem Proteinfutter.

Die Wirkstoffe können somit praktisch in allen Bereichen der Tierernährung als Mittel zur Reduzierung des Fettansatzes sowie zur Einsparung von Futtereiweiß verwendet werden.

Die Wirksamkeit der Wirkstoffe ist heirbei weitgehend unabhängig von der Art und dem Geschlecht der Tiere. Besonders wertvoll erweisen sich die Wirkstoffe bei Tierarten, die überhaupt oder in bestimmten Lebensabschnitten zu stärkerer Fetteinlagerung neigen.

## Herstellungsbeispiele

### Beispiel 1

N-Benzyl-1,5-didesoxy-1,5-imino-D-glucit XVII

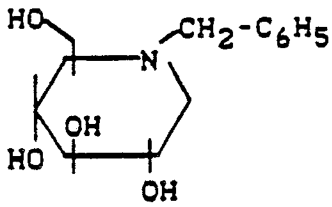

163,2 g (1,0 mol) Desoxynojirimycin werden in 2,5 1 Dimethylformamid gelöst. Der Lösung werden 76,0 g (0,55 mol) Kaliumcarbonat zugesetzt. Nach Abkühlung auf 0° C tropft man während 30 Minuten 188,1 g (1,1 mol) Benzylbromid zu. Nach etwa 120 Min. ist das Ausgangsprodukt vollständig umgesetzt. Das Produkt wird in situ weiterverarbeitet. Ein kleiner Teil wird zur Charakterisierung abgetrennt, nach Entfernung des Lösungsmittels säulenchromatographisch an Kieselgel im System Chloroform/Methanol = 4:1 gereinigt und als kristalliner Feststoff erhalten.
Fp.: 181° C $[\alpha]_D^{20}$ = -48,3° (Methanol)

Beispiel 2

N-Benzyl-4,6-O-isopropyliden-1,5-didesoxy-1,5-imino-D-glucit XVIII

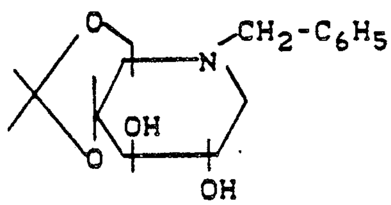

Dem Reaktionsgemisch aus Beispiel 1 wird nach Abfiltrieren des Bodenkörpers soviel p-Toluolsulfonsäure zugesetzt, bis sich ein pH von 2,0 einstellt. Sodann wird die Mischung aus 208 g (2 mol) Dimethoxypropan und 144 g (2 mol) 2-Methoxy-propan zugesetzt. Nach 2 Stunden Reaktionszeit bei 30° C wird das Reaktionsende durch Dünnschichtchromatographie in Toluol:Ethanol = 3:1 festgestellt. Die Aufarbeitung erfolgt durch mehrstündiges Rühren mit einer Suspension von 210 g Natriumhydrogencarbonat in 100 ml Wasser. Nach Verdampfen des Lösungsmittels wird der Rückstand mit Chloroform digeriert, die Chloroformphase gewaschen, über Natriumsulfat getrocknet und nach Verdampfen des Lösungsmittels der Rückstand aus Essigester/Petrolether umkristallisiert.
Ausbeute: 249,3 g
Fp.: 117° C $[\alpha]_D^{20}$ = -100,3° (Methanol)

Beispiel 3

N-Benzyl-4,6-O-isopropyliden-2,3-di-O-mesyl-1,5-didesoxy-1,5-imino-D-glucit XIX

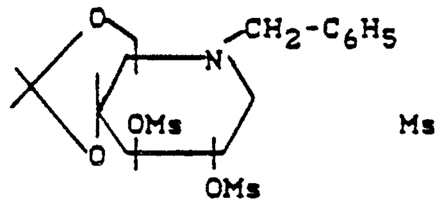

Ms = Mesyl

240 g (0,81 mol) des Produktes aus Beispiel 2 werden in 2000 ml Aceton und 300 ml Triethylamin (2,1 mol) gelöst. Hierzu wird langsam unter Eiskühlung eine Lösung von 138 ml (1,8 mol) Methansulfonsäurechlorid in 600 ml Aceton getropft. Anschließend wird die Kühlung entfernt und der Ansatz über Nacht bei Raumtemperatur gerührt. Nach vollständiger Umsetzung (DC Toluol/Aceton 4:1) wird zunächst von den augefallenen Salzen abfiltriert, sodann überschüssiges Mesylchlorid mit wenig Eis hydrolysiert und der Ansatz am Rotationsverdampfer eingeengt. Der kristalline Rückstand wird in Methylenchlorid aufgenommen, mit Wasser gewaschen, die organische Phase wird über $Na_2SO_4$ getrocknet und eingeengt.

Das Produkt ist für die weitere Umsetzung sauber genug (≥97%), kann aber aus Essigester/Petrolether umkristallisiert werden.

Ausbeute: 354 g (97% der Theorie)

Fp.: 162° C $[\alpha]_D^{20}$ = -44,8 $CHCl_3$

Beispiel 4

N-Benzyl-4,6-O-isopropyliden-1,2,3,5-tetradesoxy-1,5-imino-D-erythro-hex-2-enit XX

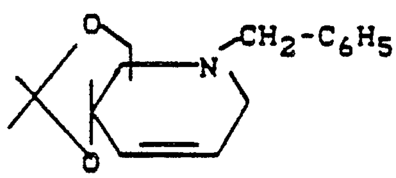

90 g (0,2 mol) des Produktes aus Beispiel 3 werden in 1600 ml Dimethylformamid gelöst, nach Zusatz von 149,9g (1,0 mol) NaI und 130,8 g (2,0 mol) Zn-Pulver 3 Stunden bei 135° C gerührt (DC: Toluol/EE 3:1). Nach dem Abkühlen wird von den Salzen abgesaugt, der gesamte Ansatz eingeengt, in $CH_2Cl_2$ aufgenommen und mehrfach mit Wasser gewaschen. Die organische Phase wird über $Na_2SO_4$ getrocknet, eingeengt und der verbleibende Sirup über Kieselgel filtriert (230-400 mesh; Laufmittel: Toluol; Toluol/Aceton 30:1).

Ausbeute: 36,3 g (70% der Theorie)

$[\alpha]_D^{20}$ = -51,4 ($CHCl_3$)

Beispiel 5

N-Benzyl-4,6-O-isopropyliden-1,5-didesoxy-1,5-imino-D-mannit III

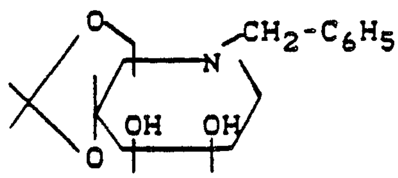

40 g (0,154 mol) des Produktes aus Beispiel 4 werden in 1200 ml Aceton, 300 ml $H_2O$ und 100 ml tert.-Butanol gelöst und nach Zugabe von 27,0 g (0,2 mol) N-Methylmorpholin-N-oxid-Hydrat und 400 mg $OsO_4$ solange gerührt, bis dünnschichtchromatographisch (Toluol/Aceton 1:1) vollständige Umsetzung beobachtet wird. Der Reaktionsansatz wird dann mit 10 ml Cyclohexen versetzt und noch 2 Stunden gerührt (Schwarzfärbung). Die Lösung wird dann über Cellite filtriert und eingeengt. Der sirupöse Rückstand wird in $CH_2Cl_2$ aufgenommen, mehrfach mit Wasser gewaschen, die organische Phase über $Na_2SO_4$ getrocknet und erneut eingeengt. Der Rückstand wird durch Flash-Chromatographie an Kieselgel gereinigt (Laufmittel:Toluol/Essigester 1:1 bis 1:3) und aus Essigester/Petrolether kristallisiert.
Ausbeute: 33,9 g (75%)
Fp.: 123-125 °C; $[\alpha]_D^{20}$ = 122,8° ($CHCl_3$)

Beispiel 6

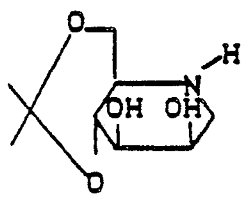

(IV)

1,5-Didesoxy-1,5-imino-4,6-O-isopropyliden-D-mannit Acetat IV

Zu einer Lösung von 72,5 g (0,25 Mol) der Verbindung III in 250 ml Methanol werden 17 ml Eisessig und 12 g 10 %ige Palladiumkohle gegeben. Die Mischung wird 5 Stunden bei 40 °C und 0,3 MPa Wasserstoffdruck gerührt. Es wird vom Palladiumkontakt abfiltriert und eingeengt. Das kristalline Produkt wird im Vakuum getrocknet. Die Ausbeute beträgt 65,8 g (100 %) m.p. 159 °C, $[\alpha]_D^{20}$ -52,5° (c 1,1; $CHCl_3$); $^1$H-NMR (300 MHz, $CD_3OD$): $\delta$ 1,37, 1,51, 1,95 (3s, 9H, $CCH_3$), 2,63 (ddd, 1H, $J_{4,5}$ 8,0 $J_{5,6a}$ = $J_{5,6b}$, 10,0 Hz, H-5), 2,96 (dd, 1H, $J_{1a,1b}$ 13,5, $J_{1a,2}$ 1,3 Hz, H-1a), 3,11 (dd, 1H, $J_{1b,2}$ 2,6 Hz, H-1b), 3,55 (dd, 1H, $J_{3,2}$ 3,0, $J_{3,4}$ 9,5 Hz, H-3), 3,79 (d, 2H, H-6a, H-6b), und 3,93-3,99 (m, 2H, H-2, H-4).
Analyse berechnet für $C_{11}H_{21}NO_6$:
C, 50,2; H, 8,0; N, 5,3
Gefunden C, 50,9; H, 7,9; N, 5,4

Beispiel 7

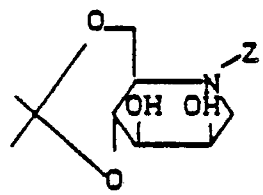

(V)

N-Benzyloxycarbonyl-1,5-didesoxy-1,5-imino-4,6-O-isopropyliden-D-mannit V

Man löst 108,6 g (0,53 Mol) der Verbindung IV in 1,5 l N,N-Dimethylformamid, in dem 86,9 g (0,63 Mol) $K_2CO_3$ suspendiert sind. Die Mischung wird auf 0° C abgekühlt und 99,5 g (0,58 Mol) Chlorameisensäurebenzylester werden hinzugetropft. Nach 30 Minuten Reaktionszeit kommt die anfänglich zu beobachtende Gasentwicklung zum Stillstand.

Durch dünnschichtchromatographische Kontrolle (Tol/EtOH = 5/1 vv, $R_f$ Produkt = 0,35) wird das Reaktionsende detektiert. Zur Aufarbeitung wird vom Bodenkörper abfiltriert und der Rückstand zweimal mit je 200 ml Dimethylformamid nachgewaschen. Das Filtrat wird im Hochvakuum eingeengt und das Rohprodukt in Chloroform aufgenommen. Die Chloroformphase wird zweimal mit gesättigter Natriumchlorid-Lösung gewaschen, über Natriumsulfat getrocknet und eingeengt. Nach Kristallisation aus Ethylacetat werden 155,6 g (0,64 Mol; 87 % der Theorie) Produkt erhalten;
mp 79-81°, $[\alpha]_D^{20}$ -8° (c 1,0, $CHCl_3$);
$^1$H-NMR (300 MHz, $CDCl_3$): $\delta$ 1,41, 1,52 (2s, 6H, C-$CH_3$), 2,58, 2,75 (2bs, 2H, C-OH), 2,92 (bd, 1H, $J_{1a,1b}$ 14,5 Hz, H-1a), 3,00 (ddd, 1H, $J_{4,5}$ 9,9, $J_{5,6a}$ 5,0, $J_{5,6b}$ 11,2 Hz, H-5), 3,57 (bddd, 1H, $J_{2,3}$ 2,5, $J_{3,4}$ 9,9, $J_{3,OH}$, 2,0 Hz, (H-3), 3,99 (dd, 1H, $J_{4,5}$ 9,9 Hz, H-4), 4,02 (bs, 1H, H-2), 4,31 (dd, 1H, $J_{6a,6b}$ 11,8 Hz, H-6a), 4,44 (dd, 1H, $J_{1b,2}$ 3,1 Hz, H-1b), 4,64 (dd, 1H, H-6b), 5,05-5,14 (2d, 2H, $J_{AB}$ 12,6 Hz, Ph-$CH_2$), und 7,28-7,43 (m, 5H, Ph-H).
Analyse berechnet für $C_{17}H_{23}NO_6$: C, 60,4; H, 6,9; N, 4,1
Gefunden: C, 61,1; H, 7,1; N, 3,8.

Beispiel 8

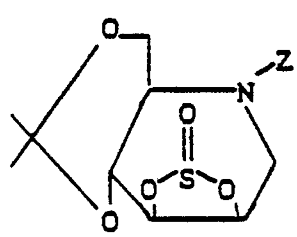

(VI)

N-Benzyloxycarbonyl-1,5-didesoxy-1,5-imino-4,6-O-isopropyliden-D-mannit-2,3-O-cyclosulfat VI

Man legt 34,3 g (102 mMol) der Verbindung V und 60 g (0,59 Mol) Triethylamin gelöst in 350 ml Ethylacetat vor. Die Lösung wird auf 0° C abgekühlt und 19 g (160 mMol), Thionylchlorid gelöst in 100 ml Ethylacetat werden langsam zugetropft. Nach 60 Minuten bei 0° C wird dünnschichtchromatographisch

(Tol/EtOH = 10/1 vv, $R_f$ Produkt = 0,5) vollständige Umsetzung nachgewiesen. Man separiert von den ausgefallenen Salzen, wäscht die organische Phase zweimal mit gesättigter Natriumchloridlösung, trocknet über Natriumsulfat und verdampft das Lösungsmittel bei 30° C. Man isoliert 38,7 g (100 mMol, 99 %) kristallines Rohprodukt, das sogleich dem nächsten Reaktionsschritt zugeführt wird. $[\alpha]_D^{20}$ -90° (c 0,5; $CHCl_3$); M + 1 384, 50 %; M + 1 + $NH_3$ 401, 90 %.

Beispiel 9

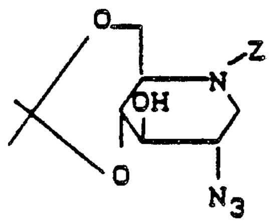

(VII)

2-Azido-N-benzyloxycarbonyl-1,2,5-tridesoxy-1,5-imino-4,6-O-isopropyliden-D-glucit VII

Man löst 45,5 g (119 mMol) der Cyclosulfit-Verbindung VI in 600 ml Dimethylformamid, in dem 13 g (267 mMol) Lithiumazid suspendiert sind. Die Suspension wird bei 90° C gerührt und der Reaktionsverlauf dünnschichtchromatographisch (Toluol/Ethylacetat 5:1 vv; $R_f$ Edukt 0,7, $R_f$ Produkt 0,9) verfolgt. Nach 8 Stunden wird aufgearbeitet. Dazu werden 75 % des Lösungsmittels im Hochvakuum bei 30° C verdampft. Der Rückstand wird in 400 ml Ethylacetat aufgenommen und die Lösung wird zweimal mit gesättigter Natriumumchloridlösung gewaschen. Nach Trocknung über Natriumsulfat wird die organische Phase eingeengt und das Rohprodukt an 500 g Kieselgel mit Toluol → Tol/Ethylacetat 10:1 als mobiler Phase chromatographiert.

Man isoliert 22,4 g (60 mMol) sirupöses Produkt. Die Ausbeute beträgt 52 %; $[\alpha]_D^{20}$ -1,2° (c 1,0; $CHCl_3$); M + 1 363; Analyse berechnet für $C_{17}H_{22}N_4O_5$: C, 56,4; H, 6,1; N, 15,5. Gefunden: C, 55,6; H, 6,3; N, 15,1.

Das O-Acetyl-Derivat ($[\alpha]_D^{20}$ -15° ; c 0,5; $CHCl_3$) liefert folgendes $^1$H-NMR Spektrum (300 MHz, $CDCl_3$): $\delta$ 1,36, 1,44, 2,12 (3s, 9H, $CCH_3$) 2,89 (dd, 1H, $J_{1a,1e}$ 13,9, $J_{1a,2}$ 10,3 Hz, H-1a), 3,15 (ddd, 1H, $J_{4,5}$ 10,2, $J_{5,6a}$ 6,1, $J_{5,6b}$ 16,1 Hz, H-5), 3,52 (ddd, 1H, $J_{1e,2}$ 4,6, $J_{2,3}$ 8,7 Hz, H-2), 3,76 (dd, 1H, $J_{3,4}$ 9,0 Hz, H-4), 4,22 (dd 1H, H-1e), 4,31-4,42 (m, 2H, H-6A, H-6b), 4,94 (dd, 1H, H-3), 5,11 (2d, 2H), $J_{A,B}$ 12,2 Hz, $PhCH_2$).

Beispiel 10

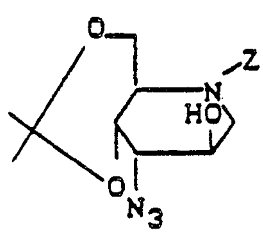

(VIIa)

3-Azido-N-benzyloxycarbonyl-1,3,5-tridesoxy-1,5-imino-4,6-O-isopropyliden-D-altrit VIIa

Man isoliert 1,8g (5 mMol) dieser Verbindung bei der chromatographischen Reinigung von Verbindung VII. Die Substanz hat einen geringfügig kleineren $R_f$-Wert als VII und wird mit einem Anteil von 8 % an der isolierten Ausbeute von VII erhalten.
$[\alpha]_D^{20}$ -37° (c 1,0; $CHCl_3$), Massenspektrum: M + 1 363; IR ($CHCl_3$): $\nu_{AS}$ $N_3$ 2122, $\nu_{C=O}$ 1707 $cm^{-1}$, Analyse berechnet für: $C_{17}H_{22}N_4O_5$:C, 56,4; H, 6,1; N 15,5.
Gefunden: C 56,9; H, 5,9; N, 15,7.

Die 2-O-acetylierte Verbindung liefert folgendes $^1$H-NMR-Spektrum (300 MHz, $CDCl_3$): 1,44, 1,54, 1,72 (3s, 9H, C-$CH_3$), 3,14 (dd, 1H, $J_{1a,1e}$ 15,0, $J_{1a,2}$ 1,5 Hz, H-1a), 3,54 (ddd, 1H, $J_{4,5}$ 10,7, $J_{5,6a}$ 5,1, $J_{5,6b}$ 10,7 Hz, H-5), 3,85 (bdd, 1H, $J_{2,3}$ 3,0 $J_{3,4}$ 3,5 Hz, H-3), 4,18 (dd, 1H, H-4), 4,20 (ddd, IH $^4J_{1e,4}$ 1,2 Hz, H-1e), 4,31 (dd, IH, $J_{6a,6b}$ 11,9 Hz, H-6a), 4,70 (dd, 1H, H-6b), 4,75 (bddd, 1H, H-2), 5,01-5,12 (2d, 2H, $J_{AB}$ 12,4 Hz, Ph-$CH_2$), 7,25-7,41 (m, 5H, Ph-H).

Beispiel 11

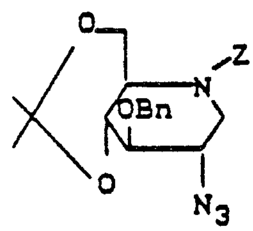

(VIII)

2-Azido-N-benzyloxycarbonyl-3-O-benzyl-1,2,5-tridesoxy-1,5-imino-4,6-O-isopropyliden-D-glucit VIII

Man löst 44,2 g (122 mMol) der Verbindung VII in 500 ml wasserfreiem Tetrahydrofuran, in dem 20,5 g (365 mMol) Kaliumhydroxid-Pulver suspendiert sind. Dem Gemenge werden 23 g (135 mMol) Benzylbromid zugesetzt. Nach 8 Stunden Reaktionszeit bei 60° C unter Ultraschall-Behandlung wird das Reaktionsende detektiert (t.l.c. Toluol/Aceton 20:1 vv; $R_f$-Edukt 0,1; $R_f$-Produkt 0,2).

Man filtriert vom Festkörper ab und verdampft das Lösungsmittel. Der Rückstand wird an 250 g Kieselgel im System Toluol → Toluol/Aceton 40:1 chromatographisch gereinigt. Es werden 44,7 g (99 mMol) der Verbindung VIII, entsprechend 81 % Ausbeute, isoliert.
$[\alpha]_D^{20}$ -15° (c 0,5, $CHCl_3$); M + 1 453; $^1$H-NMR (300 MHz, $CDCl_3$): $\delta$ 1,43, 1,49 (2s, 6H, $CCH_3$), 2,69 (dd, 1H, $J_{1a,1e}$ 13,7, $J_{1a,2}$ 10,4 Hz, H-1a), 3,07 (ddd, 1H, $J_{4,5}$ 10,2 $J_{5,6a}$ 5,0, $J_{5,6b}$ 10,7 Hz, H-5), 3,41 (dd, 1H, $J_{2,3}$ = $J_{3,4}$ 8,5 Hz, H-3), 3,48 (ddd, 1H, $J_{1e,2}$4,8 Hz, H-2), 3,83 (dd, 1H, H-4), 4,17 (dd, 1H, H-1e), 4,28 (dd, 1H, $J_{6a,6b}$ 11,8, H-6a), 4,46 (dd, 1H, H-6b), 4,73-4,89 (2d, 2H, $J_{AB}$ 11,0 Hz, Ph-$CH_2$), 5,03-5,13 (2d, 2H, $J_{AB}$ 12,2 Hz, Ph-$CH_2$). Analyse berechnet für $C_{24}H_{28}N_4O_5$:C, 63,7; H 6,2;N, 12,4;
Gefunden: C, 64,2; H, 6,6; N, 12,1.

Beispiel 12

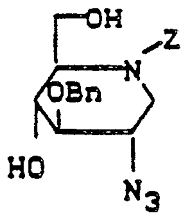

(IX)

2-Azido-N-benzyloxycarbonyl-3-O-benzyl-1,2,5-tridesoxy-1,5-imino-D-glucit IX

Es werden 44,7 g (99 mMol) der Verbindung VIII in 500 ml 50 %iger Essigsäure gelöst, der 10 % Dichlormethan zugesetzt werden. Man setzt der Mischung soviel konzentrierte Chlorwasserstoffsäure zu, bis pH 3 erreicht wird. Nach 90 Minuten bei 60° C ist die Umsetzung vollständig (t.l.c. Toluol/Ethylacetat 1:1, $R_f$-Edukt 0,8, $R_f$-Produkt 0,3).

Man setzt soviel Natriumhydrogencarbonat zu, bis pH 4,5 eingestellt ist und verdampft das Lösungsmittel. Der Rückstand wird in 200 ml Chloroform/$H_2O$ 1:1 vv aufgenommen. Die Phasen werden getrennt, die wäßrige Phase erneut mit Chloroform extrahiert und die vereinigten organischen Phasen über Natriumsulfat getrocknet.

Nach Verdampfen des Lösungsmittels isoliert man 38 g (92 mMol, 98 %) der Verbindung IX. $[\alpha]_D^{20}$ + 32°, (c 1,0: $CHCl_3$), M + 1 413.

Analyse berechnet für $C_{21}H_{24}N_4O_5$: C, 61,2; H, 5,9; N, 13,6.

Gefunden: C, 59,3; H, 5,3; N, 13,9.

Beispiel 13

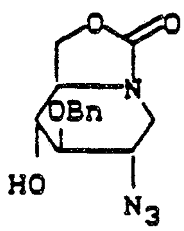

(X)

2-Azido-N-6-O-carbamoyl-3-O-benzyl-1,2,5-tridesoxy-1,5-imino-D-glucit X

In 200 ml Dimethylformamid/$H_2O$ 9:1 werden 41 g (99 mMol) der Verbindung IX gelöst. Man setzt 27,6 g (200 mMol) Kaliumcarbonat zu und rührt bei 60° C. Nach 8 Stunden ist die Umsetzung vollständig. Das Produkt zeigt dünnschichtchromatographisch einen geringfügig kleineren $R_f$-Wert als das Edukt (Hexan/Ethylacetat 1:3 vv). Zur Aufarbeitung wird von den Salzen abfiltriert, das Lösungsmittel abdestilliert, der Rückstand in Ethylacetat aufgenommen und die organische Phase mit gesättigter Natriumchloridlösung gewaschen und über Natriumsulfat getrocknet. Das Produkt kristallisiert nach Verdampfen des Lösungsmittels. Die Ausbeute beträgt 24, 7 g (81 mMol, 82 % der Theorie).

M.p. 110°, $[\alpha]_D^{20}$ -27° (c 0,5; $CHCl_3$). M + 1 + $NH_3$ 322, 100 %. IR (KBr): $\nu_{AS}N_3$ 2126, $\nu_{C=O}$ 1718 $cm^{-1}$.

Analyse berechnet für $C_{14}H_{16}N_4O_4$: C, 55,3; H, 5,3; N, 18,4.

Gefunden: C, 54,1; H, 5,6; N, 18,7.

Das O-Acetyl-Derivat liefert folgendes $^1$H-NMR-Spektrum (300 MHz, $CDCl_3$) : δ 1,98 (s, 3H, $CCH_3$), 2,70 (dd, 1H, $J_{1a,1e}$ 13,4, $J_{1e,2}$ 11,0 Hz, H-1e), 3,48 (dd, 1H, $J_{2,3}$ = $J_{3,4}$ 9,5 Hz, H-3), 3,55-3,67 (m, 2H, H-2, H-5), 4,09 (dd, 1H, $J_{1a,2}$ 5,6 Hz, H-1a), 4,22 (dd, 1H, $J_{6a,6b}$ 9,1, $J_{6a,5}$ 4,8 Hz, H-6a), 4,32 (dd, 1H, $J_{6b,5}$ 7,9 Hz, H-6b), 4,69, 4,89 (2d, 2H, $J_{AB}$ 11,1 Hz, Ph-$CH_2$, 4,92 (dd, 1H, $J_{4,5}$ 9,5 Hz, H-4).

Beispiel 14

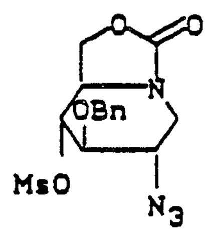

(XI)

2-Azido-N,6-O-carbamoyl-3-O-benzyl-1,2,5-tridesoxy-1,5-imino-4-O-mesyl-D-glucit XI

Man löst 22,7 g (75 mMol) der Verbindung X in 250 ml Aceton, kühlt auf 0° C ab und gibt erst 22,8 g (225 mMol) Triethylamin und dann langsam 14,3 g (125 mMol) Methansulfonsäurechlorid gelöst in 50 ml Aceton hinzu. Nach 30 Minuten Reaktionszeit ist die Umsetzung vollständig (t.l.c. Toluol/Ethylacetat 1:1, $R_f$-Edukt 0,2, $R_f$-Produkt 0,4). Man saugt von den ausgefallenen Salzen ab, verdampft das Lösungsmittel, nimmt in Ethylacetat auf, wäscht die organische Phase mit gesättigter Natriumchloridlösung, trocknet über Natriumsulfat und verdampft das Lösungsmittel. Es werden 23,2 g (61 mMol, 81 % der Theorie) kristalline Substanz isoliert. Mp 173°, $[\alpha]_D^{20}$ +15° (c 0,9; $CHCl_3$) M + 1 + $NH_3$ 400, 60 %; $^1$H-NMR (300 MHz, $CDCl_3$): δ 2,76 (dd, 1H, $J_{1a,1e}$ 13,7, $J_{1a,2}$ 11,0 Hz, H-1a), 2,82 (s, 3H, C-$CH_3$), 3,52 (dd, 1H, $J_{2,3}$ = $J_{3,4}$ 9,5 Hz, H-3), 3,60 (dd, 1H, $J_{1e,2}$ 5,4 Hz, H-2), 3,79 (ddd, $J_{4,5}$ 9,3, $J_{5,6a}$ 7,8, $J_{5,6b}$ 4,8 Hz, H-5), 4,14 (dd, 1H, H-1e), 4,39 (dd, 1H, H-4), 4,42 (dd, 1H, $J_{6a,6b}$ 9,7 Hz, H-6a), 4,56 (dd, 1H, H-6b), 4,73, 5,04 (2d, 2H, $J_{AB}$ 10,9 Hz, Ph-$CH_2$).
Analyse berechnet für $C_{15}H_{18}N_4O_6S$: C, 47,1: H, 4,7; N, 14,6
Gefunden: C, 46,8; H, 4,9; N, 14,1.

Beispiel 15

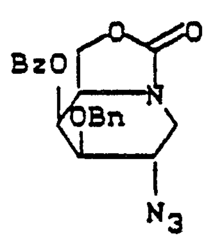

(XII)

2-Azido-N,6-O-carbamoyl-4-O-benzoyl-3-O-benzyl-1,2,5-tridesoxy-1,5-imino-D-galaktit XII

Man löst 21,2 g (52 mMol) der Verbindung XI in 350 ml wasserfreiem Dimethylformamid und setzt 8,0 g (62 mMol) Lithiumbenzoat zu. Die Mischung wird 72 Stunden unter Rühren auf 100 °C erhitzt. Dünnschichtchromatographisch kann dann vollständige Umsetzung nachgewiesen werden (t.l.c. Ethylacetat/Hexan 2:1 vv; $R_f$-Edukt 0,35, $R_f$-Produkt 0,30). Nach dem Verdampfen des Lösungsmittels wird der Rückstand mehrfach mit heißem Ethylacetat digeriert. Aus den vereinigten Eluaten werden nach dem Einengen 20 g (49 mMol, 94 % der Theorie) Produkt isoliert. Mp 161 ° ; $[\alpha]_D^{20}$ +112 ° , (c 0,4; $CHCl_3$); Massenspektrum M + 1 + $NH_3$ 426, 60 %; IR (KBr): $\nu_{AS}N_3$ 2115, $\nu_{c=o}$ 1774, 1713 $cm^{-1}$; $^1$H-NMR (300 MHz, $CDCl_3$): $\delta$ 2,73 (dd, 1H, $J_{1a,1e}$ 13,8, $J_{1a,2}$ 11,3 Hz, H-1a), 3,58 (dd, 1H, $J_{2,3}$ 9,3, $J_{3,4}$ 2,5 Hz, H-3), 3,91 (ddd, 1H, $J_{1e,2}$ 6,2 Hz, H-2), 2,96 (ddd, 1H, $J_{4,5}$ 2,0 $J_{5,6a}$ 3,2, $J_{5,6b}$ 8,9 Hz, H-5), 4,1 (dd, 1H, $J_{6a,6b}$ 9,2 Hz, H-6a), 4,23 (dd, 1H, H-1e), 4,38 (dd, 1H, H-6b), 4,59, 4,84 (2d, 2H, $J_{AB}$ 11,4 Hz, Ph-$CH_2$) und 7,2-8,1 (m, 10H 2 x Ph).

Analyse berechnet für $C_{21}H_{20}N_4O_5$: C, 61,8; H, 4,9; N, 13,7;

Gefunden: C, 62,3; H, 4,4; N, 13,4.

Beispiel 16

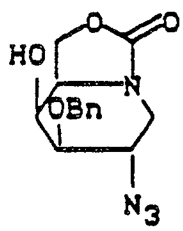

(XIII)

2-Azido-N,6-O-carbamoyl-3-O-benzyl-1,2,5-tridesoxy-1,5-imino-D-galaktit XIII

In 750 ml einer Mischung von Methanol/Dichlormethan 10:1 vv werden 20 g (49 mMol) der Verbindung XII gelöst. Man versetzt mit 5 ml einer 10 N-Natriumhydroxid-Lösung und rührt bei 40 °C. Nach 8 Stunden wird vollständige Umsetzung erreicht (t.l.c. Ethylacetat/Hexan 2:1 vv, $R_f$-Edukt 0,35, $R_f$-Produkt 0,15). Es wird mit 1- N-Chlorwasserstoffsäure neutralisiert und das Lösungsmittel verdampft. Der Rückstand wird in $CHCl_3/H_2O$ 1:1 aufgenommen und die organische Phase über Natriumsulfat getrocknet und eingeengt. Man isoliert 14,0 g (46 mMol, 94 % der Theorie). Mp 159 °, $[\alpha]_D^{20}$ -16 ° (c 0,7, $CHCl_3$). Massenspektrum: M + 1 305, 40 %; M + 1 + $NH_3$ 322, 50 %; IR (KBr): $\nu_{AS}$ $N_3$ 2120, $\nu_{c=o}$ 1747 $cm^{-1}$, Analyse berechnet für $C_{14}H_{16}N_4O_4$: C, 55,3; H, 5,3; N, 18.4.

Gefunden: C, 55,9; H, 5,3; N, 18,8.

Beispiel 17

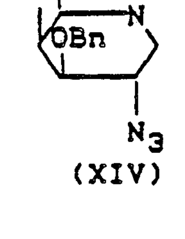

(XIV)

2-Azido-3-O-benzyl-1,2,5-tridesoxy-1,5-imino-D-galaktit XIV

Man löst 8,05 g (28,9 mMol) der Verbindung XIII in 150 ml einer Mischung aus Methanol/$H_2O$, 4:1 vv und setzt 68,3 g $Ba(OH)_2$ x 8 $H_2O$ (216 mMol) zu. Es wird 8 Stunden zum Rückfluß erhitzt (t.l.c. Toluol/Ethanol 3:1 vv, $R_f$-Edukt 0,5, $R_f$-Produkt 0,15). Die Bariumsalze werden durch Zugabe von Trockeneis gefällt, abzentrifugiert und vom Überstand getrennt. Der Festkörper wird noch mehrfach in Methanol resuspendiert und abzentrifugiert. Die vereinigten Überstände werden zur Trockene eingeengt. Man isoliert 6,4 g (22,8 mMol, 79 % der Theorie) Produkt.
Analyse berechnet für $C_{13}H_{18}N_4O_3$: C, 56,1; H, 6,5; N, 20,1;
Gefunden: C, 55,4; H, 6,1; N, 19,8.
$^1$H-NMR (300 MHz, $CD_3OD$): $\delta$ 2,31 (dd, 1H, $J_{1a,1e}$ 12,6) $J_{1a,2}$ 11,2 Hz, H-1a), 2,58 (ddd, 1H, $J_{4,5}$ 1,1, $J_{5,6a}$ = $J_{5,6b}$ = 6,6 Hz, H-5), 3,11 (dd, 1H, $J_{1e,2}$ 5,5 Hz, H-1e), 3,35 (dd, 1H, $J_{2,3}$ 9,7, $J_{3,4}$ 2,9 Hz, H-3), 6,9 (d, 2H, H-6a, H-6b), 3,75 (ddd, 1H, H-2), 4,19 (dd, 1H, H-4), 4,60, 4,78 (2d, 2H, $J_{AB}$ 11,6 Hz, $PhCH_2$) und 7,2-7,5 (m, 5H, Ph).

Beispiel 18

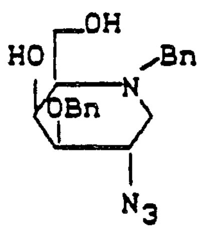

(XV)

2-Azido-N-benzyl-3-O-benzyl-1,2,5-tridesoxy-1,5-imino-D-galaktit XV

Es werden 5,0 g (10 mMol) der Verbindung XIV in 30 ml Dimethylformamid, in dem 1,4 g (10 mMol) Kaliumcarbonat suspendiert sind, gelöst. Man kühlt auf 0° C und tropft dem Gemenge 3,4 g (20 mMol) Benzylbromid langsam zu. Dann wird die Kühlung entfernt. Nach zweistündigem Rühren bei Raumtemperatur ist die Umsetzung vollständig (t.l.c. Toluol/Ethanol 3:1 vv, $R_f$-Edukt 0,15 $R_f$-Produkt 0,45). Es werden 70 ml Ethylacetat zugesetzt, dann wird von den Salzen abgesaugt und das Lösungsmittel verdampft. Man isoliert 6,3 g (17 mMol, 94 % der Theorie) Produkt. $[\alpha]_D^{20}$ -8,5° (c 1,0; $CHCl_3$), $M^{+1}$ 369, Analyse berechnet für $C_{20}H_{24}N_4O_3$: C, 65,2; H, 6,6; N, 15,2. Gefunden: C, 64,6; J, 6,9; N, 14,9.
Das di-O-Acetyl-Derivat liefert folgendes $^1$H-NMR-Spektrum (300 MHz, $CDCl_3$): $\delta$ 2,07 (dd, 1H, $J_{1a,1e}$ 12,2, $J_{1a,2}$ 5,4 Hz, H-1a), 2,08, 2,14 (2s, 6H, C-$CH_3$), 2,91 (ddd, 1H, $J_{4,5}$ 3,0, $J_{5,6a}$ 6,5 Hz, $J_{5,6b}$ 5,9 Hz, H-5), 2,98 (dd, 1H, $J_{1e,2}$ 4,4 Hz, H-1e) 3,38 (dd, 1H, $J_{2,3}$ 8,9, $J_{3,4}$ 3,1 Hz, H-3), 3,46 (d, 1H, $J_{AB}$ 13,9 Hz, Ph-$CH_2$), 3,75 (ddd, 1H, H-2), 3,92 (d, 1H, Ph-$CH_2$), 4,24 (dd, 1H, $J_{6a,6b}$ 11,6 Hz, H-6a), 4,38 (dd, 1H, H-6b), 4,50, 4,75 (2d, 1H, $J_{AB}$ 11,1 Hz, Ph-$CH_2$), 5,61 (dd, 1H, H-4) und 7,22-7,42 (m, 10H, Ph-H).

Beispiel 19

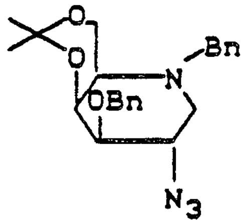

(II)

2-Azido-N-benzyl-3-O-benzyl-1,2,5-tridesoxy-1,5-imino-4,6-O-isopropyliden-D-galaktit II

Es werden 6 g (16 mMol) der Verbindung XV in 60 ml einer Mischung aus Dimethoxypropan und Dimethylformamid 5:1 vv gelöst. Man setzt tropfenweise soviel Methansulfonsäure zu, bis pH 2,5 erreicht wird und rührt bei 40° C. Nach 2 Stunden ist die Umsetzung vollständig (t.l.c. Toluol/Ethanol 10:1 vv, $R_f$-Edukt 0,1, $R_f$-Produkt 0,5). Nach Zugabe von 20 ml Triethylamin wird eingeengt. Der Rückstand wrid in Ethylacetat aufgenommen, mehrfach mit gesättigter Natriumchloridlösung extrahiert und die organische Phase über Natriumsulfat getrocknet. Nach dem Verdampfen des Lösungsmittels isoliert man 6,3 g (15 mMol, 96 % der Theorie) kristallines Produkt. Mp. 83°, $[\alpha]_D^{20}$ -0,5° (c 1,2, $CHCl_3$), Massenspektrum: M + 1 409 95 %; IR $\nu_{AS}N_3$ 2113 $cm^{-1}$. $^1H$-NMR (300 MHz, $CDCl_3$): $\delta$ 1,46, 1,54 (2s, 6H, C-$CH_3$), 1,89 (dd, 1H, $J_{1a,1e}$ = $J_{1a,2}$ 11,3 Hz, H-1a), 2,14 (bddd, 1h, H-5), 2,98 (dd, 1H, $J_{1e,2}$ 4,8 Hz, H-1e), 3,24 (dd, 1H, $J_{2,3}$ 9,9, $J_{3,4}$ 3,6 Hz, H-3), 3,33 (d, 1H, $J_{AB}$ 14,1 Hz, Ph-$CH_2$-N) 3,92 (dd, 1H, $J_{6a,6b}$ 12,6, $J_{6a,5}$ 3,1 Hz, H-6a), 3,98 (d, 1H, Ph-$CH_2$N), 3,98 (ddd, 1H, H-2), 4,14 (dd, 1H, $J_{6b,5}$ 2,60 Hz, H-6b), 4,21 (dd, 1H, $J_{4,5}$ 2,1 Hz, H-4), 4,71 (s, 2H, Ph-CH2) und 7,2-7,5 (m, 10H, Ph-H).
Analyse berechnet für $C_{23}H_{28}N_4O_3$: C, 67,6; H, 6,9; N, 13,7.
Gefunden: C, 68,1; H, 7,2; N, 13,5.

Beispiel 20

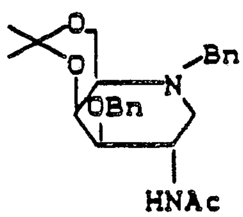

(Ia)

2-N-Acetyl-N-benzyl-3-O-benzyl-1,2,5-tridesoxy-1,5-imino-4,6-O-isopropyliden-D-galaktit Ia

In einer Mischung aus 70 ml Dioxan und 3,6 ml Eisessig werden 6,0 g (14,7 mMol) der Verbindung II gelöst. Die Lösung wird in einen Autoklaven verbracht. Man setzt 1 g 10 %ige Palladiumkohle zu und hydriert 60 Minuten bei 2 MPa und 40° C. Dünnschichtchromatographisch kann dann vollständige Umsetzung nachgewiesen werden (t.l.c. Toluol/Ethanol/Triethylamin 20:4:1 vvv; $R_f$-Edukt 0,8; $R_f$-Produkt 0,4, Ninhydrin positiv). Es wird vom Katalysator abfiltriert, das Lösungsmittel verdampft und der Rückstand in 30 ml einer Mischung aus Pyridin und Acetanhydrid 2:1 vv aufgenommen. Nach 4 Stunden bei 40 °C wird vollständige Umsetzung erreicht (t.l.c. Toluol/Ethanol 5:1 vv, $R_f$-Edukt 0,13, Ninhydrin positiv, $R_f$-Produkt

0,22 Ninhydrin negativ).

Die Reagenzien werden am Rotationsverdampfer abdestilliert und der Rückstand wird in $CHCl_3$ aufgenommen, mit gesättigter Natriumchloridlösung gewaschen und die organische Phase über Natriumsulfat getrocknet und eingeengt. Das Rohprodukt wird an 500 g Kieselgel im System Dichlormethan/Ethanol 80:1 → 20:1 +0,1 % Triethylamin chromatographisch gereinigt. Man isoliert 4,9 g (11,5 mMol, 78,2 % der Theorie) sirupöses Produkt; $[\alpha]_D^{20}$ +41° (c 1,2, $CHCl_3$).

Massenspektrum: M + 1 425 100 %; IR ($CHCl_3$): $\nu_{N\text{-}H}$ 3435, $\nu_{C=O}$ 1730, $\nu_{CNH}$ 1514 $cm^{-1}$; $^1$H-NMR (300 MHz $C_6D_6$): $\delta$ 1,28, 1,43, 1,56 (3s, 9H, $CCH_3$), 1,75 (bs, 1H, H-5), 2,20 (dd, 1H, $J_{1a,1e}$ = $J_{1a,2}$ 10,9 Hz, H-1a), 3,18 (d, 1H, $J_{AB}$ 13,7 Hz, Ph-$CH_2$), 3,21 (dd, 1H, $J_{1e,2}$ 4,30 Hz, H-1e), 3,53 (dd, 1H, $J_{2,3}$ 10,8, $J_{3,4}$ 3,2 Hz, H-3), 3,54 (dd, 1H, $J_{5,6a}$ 3,6, $J_{6a,6b}$ 12,6 Hz, H-6a), 3,77 (d, 1H, Ph-$CH_2$), 3,94 (dd, 1H, $J_{5,6b}$ 2,1 Hz, H-6b), 4,04 (bs, 1H, $J_{4,5}$ 2,0 Hz, H-4), 4,28 (dddd, 1H, H-2) 4,41, 4,60 (2d, 2H, $J_{AB}$ 12,0 Hz, Ph-$CH_2$), und 4,69 (bd, 1H, $J_{NH,2}$ 6,5 Hz, NH).

Analyse berechnet für: $C_{25}H_{32}N_2O_4$: C, 70,7; H, 7,6; N, 6,6.

Gefunden: C, 71,3; H, 7,9; N 6,4/

Beispiel 21

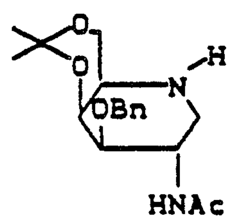

(Ib)

2-N-Acetyl-3-O-benzyl-1,2,5-tridesoxy-1,5-imino-4,6-O-isopropyliden-D-galaktit Ib

Man löst 0,5 g (1,2 mMol) der Verbindung Ia in 5 ml Dioxan, dem 0.1 g 10 %ige Palladiumkohle und 0,01 ml Essigsäure zugesetzt werden. Es wird 6 Stunden bei 1,0 MPa hydriert (t.l.c. Dichlormethan/Ethanol 5:1 vv, $R_f$-Edukt 0,7, $R_f$-Produkt 0,1). Es wird vom Katalysator abfiltriert und das Lösungsmittel abdestilliert. Der Rückstand wird an 50 g Kieselgel im System Dichlormethan/Ethanol/Triethylamin 10/1/0,01 chromatographisch gereinigt. Man isoliert 0,3 g (0,9 mMol, 75 % der Theorie) reines Produkt. $[\alpha]_D^{20}$ +95° (c 0,95; $CHCl_3$), Massenspektrum: M + 1 335, 100 %, $^1$H-NMR (300 MHz, $CD_3OD$): $\delta$ 1,42 (s, 6H, $CCH_3$); 1,93 (s, 3H, $CCH_3$) 2,43 (dd, 1H, $J_{1a,1e}$ 13,3, $J_{1a,2}$11,6 Hz, H-1a), 2,53 (bs, 1H, H-5), 3,16 (dd, 1H, $J_{1e,2}$5,0 Hz, (H-1e), 3,49 (dd, 1H, $J_{2,3}$ 10,4 $J_{3,4}$ 3,0 Hz, H-3), 3,67 (dd, 1H, $J_{6a,6b}$ 11,9, $J_{5,6a}$ 1,40 Hz, H-6a), 4,16 (dd, 1H, $J_{5,6b}$ 1,9 Hz, H-6b), 4,17 (ddd, 1H, H-2), 4,46 (bdd, 1H, $J_{4,5}$ 2,5 Hz, H-4), 4,56, 4,66 (2d, 2H, $J_{AB}$ 12,0 Hz, $PhCH_2$), und 7,2-7,4 (m, 5H, Ph-H).

Analyse berechnet für $C_{18}H_{26}N_2O_4$: C, 64,7; H, 7,8; N, 8,4.

Gefunden: C, 64,2; H, 7,7; N, 8,3.

Beispiel 22

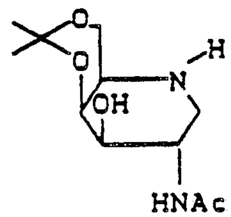

(Ic)

2-N-Acetyl-1,2,5-tridesoxy-1,5-imino-4,6-O-isopropyliden-D-galaktit Ic

In 50 ml Eisessig werden 1,4 g (3,29 mMol) der Verbindung Ib gelöst und mit 1,4 g 10 %iger Palladiumkohle versetzt. Die Mischung wird in einen Autoklaven übergeführt und 8 Stunden bei 4,0 MPa und 40° C hydriert (t.l.c. Chloroform/Methanol/Ammoniaklösung 4/3/1 vvv, $R_f$-Edukt 0,95, $R_f$-Produkt 0,8). Es wird vom Katalysator abfiltriert, das Lösungsmittel im Vakuum verdampft, der Rückstand in Methanol aufgenommen und mit Ionenaustauscher IRA 400 pH 8 eingestellt. Nach Abtrennen des Ionenaustauschers wird das Lösungsmittel verdampft und der Rückstand an 50 g Kieselgel RP-18 im System $H_2O$/Acetonitril 9/1 vv chromatographisch gereinigt. Man isoliert 0,65 g (2,7 mMol, 81 % der Theorie) Produkt, $[\alpha]_D^{20}$ + 81° (c 1,0, Methanol), Massenspektrum: M + 1 245, 100 %; IR (KBr): $\nu_{(N\text{-}H)trans}$ 3308 $\nu_{C=O}$ 1674, $\nu_{C\text{-}N}$, $\delta$ CNH 1510 $cm^{-1}$; $^1H$-NMR (300 MHz, $CD_3OD$, Acetic acid salt): $\delta$ 1,37, 1,42, 1,89 (3s, 9H, C-$CH_3$), 2,23 (dd, 1H, $J_{1a,1e}$ 13,6, $J_{1a,2}$ 11,5 Hz, H-1a), 2,40 )bs, 1H, H-5), 3,05 (dd, 1H, $J_{1e,2}$ 4,8 Hz, H-1e), 3,39 (dd, 1H, $J_{2,3}$ 10,5, $J_{3,4}$ 3,1 Hz, H-3), 3,39 (dd, 1H, $J_{6a,6b}$ 12,3, $J_{6a,5}$ 1,7 Hz, H-6a), 3,93 (ddd, 1H, H-2), 4,10 (dd, 1H, $J_{6b,5}$ 2,2 Hz, H-6b) und 4,15 (dd, 1H, $J_{4,5}$ 1,1 Hz, H-4).

Analyse berechnet für $C_{11}H_{20}N_2O_4$: C, 54,1; H, 8,3; N, 11,5.

Gefunden: C, 54,3; H, 7,9; N, 11,6.

Beispiel 23

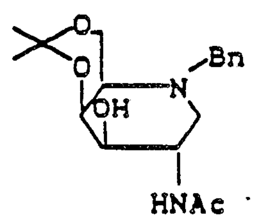

(Id)

2-N-Acetyl-N-benzyl-1,2,5-tridesoxy-1,5-imino-4,6-O-isopropyliden-D-galaktit Id

In 5 ml Dimethylformamid werden 0,24 g (1,0 mMol) der Verbindung Ic gelöst. Man kühlt ab auf 0° C, setzt 88 mg (0,6 mMol) Kaliumcarbonat und 190 mg (1,1 mMol) Benzylbromid zu und läßt unter Rühren 8 stunden bei Raumtemperatur reagieren (t.l.c. Toluol/Ethanol 1/3 vv, $R_f$-Edukt 0,1, $R_f$-Produkt 0,7). Man filtriert vom Salz ab und verdampft das Lösungsmittel. Der Rückstand wird in Chloroform aufgenommen und die Lösung mit gesättigter Natriumchloridlösung gewaschen und über Natriumsulfat getrocknet. Nach dem Verdampfen des Lösungsmittels wird die Substanz an Kieselgel 60 im System Toluol → Toluol/Ethanol 1:1 vv chromatographisch gereinigt. Es werden 0,29 g (0,86 mMol, 86 % der Theorie) Produkt isoliert; $[\alpha]_D^{20}$

+18° (c 1,0; $CHCl_3$); Massenspektrum: M + 1 335, 100 %; IR ($CHCl_3$): $\nu_{(N-H)trans}$ 3442, $\nu_{C=O}$ 1672, $\nu_{C-N}$, $\delta$ CNH 1515 $cm^{-1}$; $^1$H-NMR (300 MHz, $D_2O$, Hydrochlorid): $\delta$ 1,45, 1,50, 1,89 (3s, 9H, $CCH_3$) 2,83 (dd, 1H, $J_{1a,1e}$ $J_{1a,2}$ 12,2 Hz, H-1a), 3,31 (bs, 1H, H-5), 3,40 (dd, 1H, $J_{1e,2}$ 4,4 Hz, H-1e), 3,64 (dd, 1H, $J_{2,3}$ 10,9, $J_{3,4}$ 3,1 Hz, H-3), 4,29 (ddd, 1H, H-2), 4,31 (dd, 1H, $J_{6a,6b}$ 12,9, $J_{6a,5}$ 1,5 Hz, H-6a), 4,40, 4,52 (2, 2H, $J_{A,B}$ 13,5 Hz, $PhCH_2$), 4,54 (dd, 1H, $J_{6b,5}$ 1 Hz, H-6b), 4,55 (dd, 1H, H-4) und 7,33-7,52 (m, 5H, Ph-H).
Analyse berechnet für $C_{18}H_{26}N_2O_4$: C, 64,7; H, 7,8; N, 8,4.
Gefunden: C, 64,3; H, 7,7; N, 8,2.

Beispiel 24

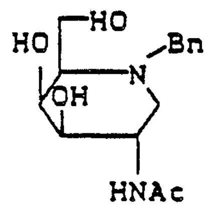

(Ie)

2-N-Acetyl-N-benzyl-1,2,5-tridesoy-1,5-imino-D-galaktit Ie

Man löst 0,14 g (0,42 mMol) der Verbindung Id in 2 ml 50 %iger Essigsäure, der soviel 1-N-Salzsäure zugesetzt wird, bis pH 3 eingestellt ist. Die Lösung wird 36 Stunden bei 60° C gerührt (t.l.c. Toluol/Ethanol 1/1 vv, $R_f$-Edukt 0,4, $R_f$-Produkt 0,1). Das Lösungsmittel wird im Hochvakuum bei 30° C verdampft und der Rückstand in Methanol aufgenommen. Man neutralisiert durch Zugabe von basischem Ionenaustauscher IRA 400, verdampft das Lösungsmittel und chromatographiert das Produkt an 50 g Kieselgel RP-18 im Systen $H_2O$/Acetonitril 9:1 → 4:1.
Es werden 0,095 g (0,32 mMol, 76 % der Theorie) Produkt isoliert: $[\alpha]_D^{20}$ +22,4° (c 0,75; Methanol), Massenspektrum: M + 1 295, 100 %; $^1$H-NMR (300 MHz, Hydrochlorid, $CH_3OD/D_2O$ 1:1 vv): $\delta$ 1,94 (s, 3H, $CCH_3$), 2,17 (bdd, 1H, $J_{1a,1e}$ 11,3 Hz, H-1a), 2,84 (bs, 1H, H-5), 2,94 (dd, 1H, $J_{1e,2}$ 4,3 Hz, H-1e), 3,51 (dd, 1H, $J_{2,3}$ 10,5, $J_{3,4}$ 3,1 Hz, H-3), 3,73 (bd, 1H, $J_{A,B}$ 13,4 Hz, $PhCH_2$), 4,01 (dd, 1H, $J_{5,6a}$ 6,0, $J_{6a,6b}$ 11,8 Hz, H-6a), 4,11 (dd, 1H, $J_{5,6b}$ 5,0 Hz, H-6b), 4,13 (m, H-4), 4,18 (m, H-2), 4,24 (bd, 1H, $PhCH_2$) und 7,40 (s, 5H, Ph-H).
Analyse berechnet für $C_{15}H_{22}N_2O_4$: C, 61,2; H, 7,5; N, 9,5.
Gefunden: C, 61,4; H, 7,3; N, 9.7.

Beispiel 25

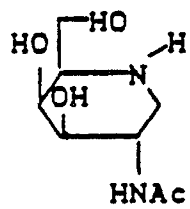

(If)

2-N-Acetyl-1,2,5-tridesoxy-1,5-imino-D-galaktit If

Verbindung Ie (0,090 g, 0,31 mMol) wird in 5 ml einer 50 %igen Lösung von Dioxan in Wasser gelöst. Der Lösung werden 0,1 g 10 %ige Palladiumkohle zugesetzt. Man hydriert 5 Stunden bei 0,3 MPa Wasserstoffdruck. Dünnschichtchromatographisch (4:3:1 Chloroform/Methanol/Ammoniaklösung. $R_f$-Produkt = 0,31) läßt sich dann kein Edukt mehr nachweisen.

Der Katalysator wird abfiltriert und das Lösungsmittel wird im Vakuum verdampft. Der Rückstand wird erneut in Wasser aufgenommen. Nach Gefriertrocknung erhält man 0,059 g (0,29 mMol, 93 % der Theorie) Produkt. $[\alpha]_D^{20}$ +37° (c = 1,0; Methanol); Massenspektrum: M + 1 205, $R_f$ 0,31 (4:2:1 Chloroform/Methanol/Ammoniaklösung).

$^1$H-NMR (300 MHz, Hydrochlorid, $D_2O$):

δ 1,9 l (s, 3H, $CCH_3$), 2,79 (dd, 1H, $J_{1a,1c}$ 12,5, $J_{1a,2}$ 12,0 Hz, H-1a), 3,28-3,41 (m, 2H, H-5, H 1c), 3,6-3,8 (m, 3H), 4,08 (bs, 1H, H-4), 4,2 (ddd, 1H, H-Z).

## Ansprüche

1. 6-Hydroxymethylenverzweigte 3-Amino-4,5-dihydroxypiperidine der allgemeinen Formel (I)

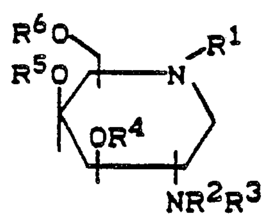

(I)

in welcher

$R^1$ - für Wasserstoff steht, oder

- für geradkettiges oder verzweigtes Alkyl mit bis zu 8 Kohlenstoffatomen steht, das ein- oder mehrfach gleich oder verschieden durch Halogen substituiert sein kann, oder
- für geradkettiges oder verzweigtes Alkenyl mit bis zu 4 Kohlenstoffatomen steht, oder
- für Aralkyl mit 7 bis 14 Kohlenstoffatomen steht, das im Aromaten ein- oder mehrfach gleich oder verschieden durch Halogen oder Alkoxy mit bis zu 4 Kohlenstoffatomen substituiert sein kann, oder
- für eine Gruppe der Formel

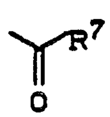

steht,

worin

$R^7$ - geradkettiges oder verzweigtes Alkyl oder Alkenyl mit bis zu 8 Kohlenstoffatomen bedeutet, oder

- geradkettiges oder verzweigtes Alkoxy mit bis zu 8 Kohlenstoffatomen bedeutet, oder

-Aralkoxy mit bis zu 10 Kohlenstoffatomen bedeutet,

$R^2$, $R^3$ - gleich oder verschieden ist und

- für Wasserstoff steht, oder
- für geradkettiges oder verzweigtes Alkyl mit bis zu 8 Kohlenstoffatomen steht, das ein- oder mehrfach gleich oder verschieden durch Halogen substituiert sein kann, oder
- für geradkettiges oder verzweigtes Alkenyl mit bis zu 6 kohlenstoffatomen steht, oder
- für Aralkyl mit 7 bis 14 Kohlenstoffatomen steht, das im Aromaten ein- oder mehrfach gleich oder verschieden durch Halogen oder durch Alkoxy mit bis zu 4 Kohlenstoffatomen substituiert sein kann, oder
- für eine Gruppe der Formel

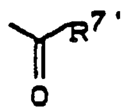

steht,

worin
$R^{7'}$ - die gleiche Bedeutung hat wie $R^7$ und mit diesem gleich oder verschieden sein kann,
$R^4$ - für Wasserstoff steht, oder
- für geradkettiges oder verzweigtes Alkyl mit bis zu 4 Kohlenstoffatomen steht, das ein- oder mehrfach gleich oder verschieden durch Halogen substituiert sein kann, oder
- für Alkenyl mit bis zu 4 Kohlenstoffatomen steht, oder
- für Aralkyl mit 7 bis 14 Kohlenstoffatomen steht, das im Aromaten ein- oder mehrfach gleich oder verschieden durch Halogen oder durch Alkoxy mit bis zu 4 Kohlenstoffatomen substituiert sein kann, oder
- für eine Gruppe der Formel

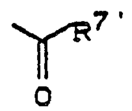

steht,

wobei
$R^{7'}$ - die oben angegebene Bedeutung hat
und
$R^5$, $R^6$ - gleich oder verschieden ist und
- für Wasserstoff steht, oder
- für geradkettiges oder verzweigtes Alkyl oder Alkenyl mit bis zu 4 Kohlenstoffatomen steht oder,
- für Aralkyl mit bis 14 Kohlenstoffatomen steht, das im Aromaten ein- oder mehrfach gleich oder verschieden durch Halogen oder Alkoxy mit bis zu 4 Kohlenstoffatomen substituiert sein kann, oder
- für eine Gruppe der Formel

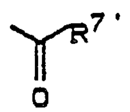

steht,

wobei
$R^{7'}$ - die oben angegebene Bedeutung hat,
oder
$R^5$, $R^6$ - gemeinsam eine Methylen-, Ethylen-, Propyliden-, Isopropyliden-, Cyclohexyliden- oder Benzylidengruppe bilden
und deren physiologisch unbedenkliche Salze.

2. 6-Hydroxymethylenverzweigte 3-Amino-4,5-dihydroxypiperidine der allgemeinen Formel (I) gemäß Anspruch 1,
in welcher
$R^1$ - für Wasserstoff steht, oder
- für Alkyl mit bis zu 6 Kohlenstoffatomen steht, das ein- oder mehrfach gleich oder verschieden durch Fluor oder Chlor substituiert sein kann, oder
- für Allyl steht, oder
- für Benzyl steht, das im Phenylring ein- oder mehrfach gleich oder verschieden durch Fluor, Chlor, Brom oder Methoxy substituiert sein kann, oder
- für eine Gruppe der Formel

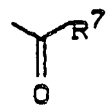 steht,

worin

$R^7$ - geradkettiges oder verzweigtes Alkyl oder Alkenyl mit bis zu 4 Kohlenstoffatomen bedeutet, oder

- geradkettiges oder verzweigtes Alkoxy mit bis zu 4 Kohlenstoffatomen bedeutet, oder
- Benzyloxy bedeutet,

$R^2$ - für Wasserstoff steht,

$R^3$ - für Wasserstoff steht, oder

- für Alkyl mit bis zu 6 Kohlenstoffatomen steht, das ein- oder mehrfach gleich oder verschieden durch Fluor oder Chlor substituiert sein kann,
- für Allyl steht, oder
- für Benzyl steht, das im Phenylring ein- oder mehrfach gleich oder verschieden durch Fluor, Chlor, Brom oder Methoxy substituiert sein kann, oder
- für eine Gruppe der Formel

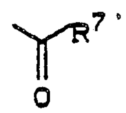 steht,

wobei

$R^{7'}$ - die gleiche Bedeutung hat wie $R^7$ und mit diesem gleich oder verschieden sein kann,

$R^4$ - für Wasserstoff, Methyl, Ethyl oder Allyl steht, oder

- für Benzyl steht, das im Phenylrest ein- oder mehrfach gleich oder verschieden durch Fluor, Chlor, Brom oder Methoxy substituiert sein kann, oder
- für eine Gruppe der Formel

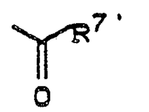 steht,

worin

$R^{7'}$ - die oben angegebene Bedeutung hat,

und

$R^5$, $R^6$ - gleich oder verschieden ist und

- für Wasserstoff, Methyl oder Ethyl steht, oder
- für Benzyl steht, das im Phenylrest ein- oder mehrfach, gleich oder verschieden durch Fluor, Chlor oder Methoxy substituiert sein kann, oder
- für eine Gruppe der Formel

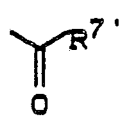 steht,

worin

$R^{7'}$ - die oben angegebene Bedeutung hat,

oder

$R^5$, $R^6$ - gemeinsam eine Methylen-, Isopropyliden- oder Cyclohexylidengruppe bilden

und deren physiologisch unbedenkliche Salze.

3. 6-Hydroxymethylenverzweigte 3-Amino-4,5-dihydroxypiperidine der allgemeinen Formel (I) gemäß Anspruch 1,
in welcher
$R^1$ - für Wasserstoff steht, oder
- für Alkyl mit bis zu 4 Kohlenstoffatomen steht, oder
- für Benzyl, 4-Chlorbenzyl, 4-Fluorbenzyl oder 4-Methoxybenzyl steht, oder
- für Acetyl, Methoxycarbonyl, Ethoxycarbonyl oder Benzyloxycarbonyl steht,
$R^2$ - für Wasserstoff steht,
$R^3$ -für Wasserstoff steht, oder
- für Alkyl mit bis zu 4 Kohlenstoffatomen steht, oder
- für Benzyl, 4-Chlorbenzyl, 4-Fluorbenzyl oder 4-Methoxybenzyl steht, oder
- für Acetyl steht,
$R^4$ - für Wasserstoff steht, oder
- für Benzyl oder 4-Methoxybenzyl steht, oder
- für Acetyl steht,
und
$R^5$, $R^6$ - gleich oder verschieden ist und
- für Wasserstoff steht, oder
- für Benzyl oder 4-Methoxybenzyl steht, oder
- für Acetyl steht,
oder
$R^5$, $R^6$ - gemeinsam eine Isopropylidengruppe bilden
und deren physiologisch unbedenkliche Salze.

4. 6-Hydroxymethylenverzweigte 3-Amino-4,5-dihydroxypiperidein der allgemeinen Formel (I)

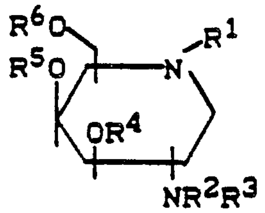

(I)

in welcher
$R^1$ - für Wasserstoff steht, oder
- für geradkettiges oder verzweigtes Alkyl mit bis zu 8 Kohlenstoffatomen steht, das ein- oder mehrfach gleich oder verschieden durch Halogen substituiert sein kann, oder
- für geradkettiges oder verzweigtes Alkenyl mit bis zu 4 Kohlenstoffatomen steht, oder
- für Aralkyl mit 7 bis 14 Kohlenstoffatomen steht, das im Aromaten ein- oder mehrfach gleich oder verschieden durch Halogen oder Alkoxy mit bis zu 4 Kohlenstoffatomen substituiert sein kann, oder
- für eine Gruppe der Formel

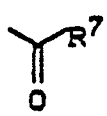

steht,

worin
$R^7$ - geradkettiges oder verzweigtes Alkyl mit bis zu 8 Kohlenstoffatomen bedeutet, oder
- geradkettiges oder verzweigtes Alkoxy mit bis zu 8 Kohlenstoffatomen bedeutet, oder
- Aralkoxy mit bis zu 10 Kohlenstoffatomen bedeutet,
$R^2$, $R^3$ - gleich oder verschieden ist und
- für Wasserstoff steht, oder
- für geradkettiges oder verzweigtes Alkyl mit bis zu 8 Kohlenstoffatomen steht, das ein- oder mehrfach gleich oder verschieden durch Halogen substituiert sein kann, oder
- für geradkettiges oder verzweigtes Alkenyl mit bis zu 6 Kohlenstoffatomen steht, oder

- für Aralkyl mit 7 bis 14 Kohlenstoffatomen steht, das im Aromaten ein- oder mehrfach gleich oder verschieden durch Halogen oder durch Alkoxy mit bis zu 4 Kohlenstoffatomen substituiert sein kann, oder
- für eine Gruppe der Formel

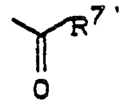

steht,

worin
$R^{7'}$ - die gleiche Bedeutung hat wie $R^7$ und mit diesem gleich oder verschieden sein kann,
$R^4$ - für Wasserstoff steht, oder
- für geradkettiges oder verzweigtes Alkyl mit bis zu 4 Kohlenstoffatomen steht, das ein- oder mehrfach gleich oder verschieden durch Halogen substituiert sein kann, oder
- für Alkenyl mit bis zu 4 Kohlenstoffatomen steht, oder
- für Aralkyl mit 7 bis 14 Kohlenstoffatomen steht, das im Aromaten ein - oder mehrfach gleich oder verschieden durch Halogen oder durch Alkoxy mit bis zu 4 Kohlenstoffatomen substituiert sein kann, oder
- für eine Gruppe der Formel

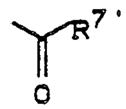

steht,

wobei
$R^{7'}$ - die oben angegebene Bedeutung hat
und
$R^5$, $R^6$ - gleich oder verschieden ist und
- für Wasserstoff steht, oder
- für geradkettiges oder verzweigtes Alkyl mit bis zu 4 Kohlenstoffatomen steht, oder
- für Aralkyl mit 7 bis 14 Kohlenstoffatomen steht, das im Aromaten ein- oder mehrfach gleich oder verschieden durch Halogen oder Alkoxy mit bis zu 4 Kohlenstoffatomen substituiert sein kann, oder
- für eine Gruppe der Formel

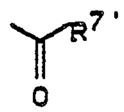

steht,

wobei
$R^{7'}$ - die oben angegebene Bedeutung hat
oder
$R^5$, $R^6$ - gemeinsam eine Methylen-, Ethylen-, Propyliden-, Isopropyliden-, Cyclohexyliden- oder Benzylidengruppe bilden
und deren physiologisch unbedenkliche Salze,
zur Verwendung bei der therapeutischen Behandlung des menschlichen oder tierischen Körpers.

5. Verfahren zur Herstellung von 6-Hydroxy-methylenverzweigten 3-Amino-4,5-dihydroxypiperidinen der allgemeinen Formel (I)

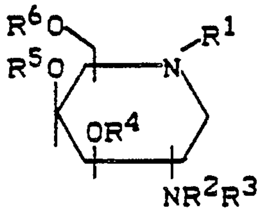

(I)

in welcher
$R^1$ - für Wasserstoff steht, oder
- für geradkettiges oder verzweigtes Alkyl mit bis zu 8 Kohlenstoffatomen steht, das ein- oder mehrfach gleich oder verschieden durch Halogen substituiert sein kann, oder
- für geradkettiges oder verzweigtes Alkenyl mit bis zu 4 Kohlenstoffatomen steht, oder
- für Aralkyl mit 7 bis 14 Kohlenstoffatomen steht, das im Aromaten ein- oder mehrfach gleich oder verschieden durch Halogen oder Alkoxy mit bus zu 4 Kohlenstoffatomen substituiert sein kann, oder
- für eine Gruppe der Formel

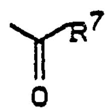

steht,

worin
$R^7$ - geradkettiges oder verzweigtes Alkyl mit bis zu 8 Kohlenstoffatomen bedeutet, oder
- geradkettiges oder verzweigtes Alkoxy mit bis zu 8 Kohlenstoffatomen bedeutet, oder
- Aralkoxy mit bis zu 10 Kohlenstoffatomen bedeutet,
$R^2$, $R^3$ - gleich oder verschieden ist und
- für Wasserstoff steht, oder
- für geradkettiges oder verzweigtes Alkyl mit bis zu 8 Kohlenstoffatomen steht, das ein- oder mehrfach gleich oder verschieden durch Halogen substituiert sein kann, oder
- für geradkettiges oder verzweigtes Alkenyl mit bis zu 6 Kohlenstoffatomen steht, oder
- für Aralkyl mit 7 bis 14 Kohlenstoffatomen steht, das im Aromaten ein- oder mehrfach gleich oder verschieden durch Halogen oder durch Alkoxy mit bis zu 4 Kohlenstoffatomen substituiert sein kann, oder
- für eine Gruppe der Formel

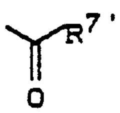

steht,

worin
$R^{7'}$ - die gleiche Bedeutung hat wie $R^7$ und mit diesem gleich oder verschieden sein kann,
$R^4$ - für Wasserstoff steht, oder
- für geradkettiges oder verzweigtes Alkyl mit bis zu 4 Kohlenstoffatomen steht, das ein- oder mehrfach gleich oder verschieden durch Halogen substituiert sein kann, oder
- für Alkenyl mit bis zu 4 Kohlenstoffatomen steht, oder
- für Aralkyl mit 7 bis 14 Kohlenstoffatomen steht, das im Aromaten ein- oder mehrfach gleich oder verschieden durch Halogen oder durch Alkoxy mit bis zu 4 Kohlenstoffatomen substituiert sein kann, oder
- für eine Gruppe der Formel

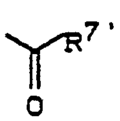

steht,

wobei
$R^{7'}$ - die oben angegebene Bedeutung hat
und
$R^5$, $R^6$ - gleich oder verschieden ist und
- für Wasserstoff steht, oder
- für geradkettiges oder verzweigtes Alkyl mit bis zu 4 Kohlenstoffatomen steht, oder
- für Aralkyl mit 7 bis 14 Kohlenstoffatomen steht, das im Aromaten ein- oder mehrfach gleich oder verschieden durch Halogen oder Alkoxy mit bis zu 4 Kohlenstoffatomen substituiert sein kann, oder
- für eine Gruppe der Formel

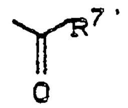

steht,

wobei
$R^{7'}$ - die oben angegebene Bedeutung hat,
oder
$R^5$, $R^6$ - gemeinsam eine Methylen-, Ethylen-, Propyliden-, Isopropyliden-, Cyclohexyliden- oder Benzylidengruppe bilden
und deren physiologisch unbedenklichen Salzen,
dadurch gekennzeichnet, daß man
Azidoverbindungen der allgemeinen Formel (II)

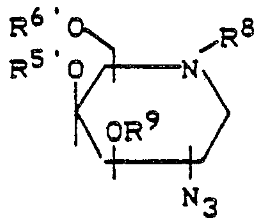

(II)

in welcher
$R^{5'}$ und $R^{6'}$ gemeinsam eine Isopropylidengruppe bilden,
$R^8$ - für eine Aminoschutzgruppe, bevorzugt für Benzyl steht,
und
$R^9$ - für eine Hydroxylschutzgruppe, bevorzugt für Benzyl, 4-Methoxybenzyl oder Allyl steht,
in inerten Lösemitteln, in Anwesenheit eines Reduktionsmittels, gegebenenfalls in Anwesenheit eines Katalysators reduziert,
gegebenenfalls Schutzgruppen abspaltet, oder neue Schutzgruppen einführt,
und gegebenenfalls anschließend mit Säure die entsprechenden Salze herstellt.

6. Arzneimittel, enthaltend 6-Hydroxy-methylenverzweigte 3-Amino-4,5-dihydroxypiperidine der allgemeinen Formel (I)

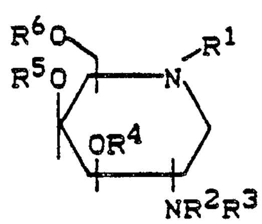

(I)

in welcher
$R^1$ - für Wasserstoff steht, oder
- für geradkettiges oder vezweigtes Alkyl mit bis zu 8 Kohlenstoffatomen steht, das ein- oder mehrfach

gleich oder verschieden durch Halogen substituiert sein kann, oder
- für geradkettiges oder verzweigtes Alkenyl mit bis zu 4 Kohlenstoffatomen steht, oder
- für Aralkyl mit 7 bis 14 Kohlenstoffatomen steht, das im Aromaten ein- oder mehrfach gleich oder verschieden durch Halogen oder Alkoxy mit bis zu 4 Kohlenstoffatomen substituiert sein kann, oder
- für eine Gruppe der Formel

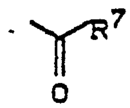 steht,

worin
$R^7$ - geradkettiges oder verzweigtes Alkyl mit bis zu 8 Kohlenstoffatomen bedeutet, oder
- geradkettiges oder verzweigtes Alkoxy mit bis zu 8 Kohlenstoffatomen bedeutet, oder
- Aralkoxy mit bis zu 10 Kohlenstoffatomen bedeutet,
$R^2$, $R^3$- gleich oder verschieden ist und
- für Wasserstoff steht, oder
- für geradkettiges oder verzweigtes Alkyl mit bis zu 8 Kohlenstoffatomen steht, das ein- oder mehrfach gleich oder verschieden durch Halogen substituiert sein kann, oder
- für geradkettiges oder verzweigtes Alkenyl mit bis zu 6 Kohlenstoffatomen steht, oder
- für Aralkyl mit 7 bis 14 Kohlenstoffatomen steht, das im Aromaten ein- oder mehrfach gleich oder verschieden durch Halogen oder durch Alkoxy mit bis zu 4 Kohlenstoffatomen substituiert sein kann, oder
- für eine Gruppe der Formel

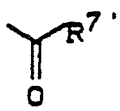 steht,

worin
$R^{7'}$ - die gleiche Bedeutung hat wie $R^7$ und mit diesem gleich oder verschieden sein kann,
$R^4$ - für Wasserstoff steht, oder
- für geradkettiges oder verzweigtes Alkyl mit bis zu 4 Kohlenstoffatomen steht, das ein- oder mehrfach gleich oder verschieden durch Halogen substituiert sein kann, oder
- für Alkenyl mit bis zu 4 Kohlenstoffatomen steht, oder
- für Aralkyl mit 7 bis 14 Kohlenstoffatomen steht, das im Aromaten ein- oder mehrfach gleich oder verschieden durch Halogen oder durch Alkoxy mit bis zu 4 Kohlenstoffatomen substituiert sein kann, oder
- für eine Gruppe der Formel

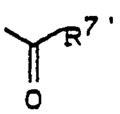 steht,

wobei
$R^{7'}$ - die oben angegebene Bedeutung hat
und
$R^5$, $R^6$ - gleich oder verschieden ist und
- für Wasserstoff steht, oder
- für geradkettiges oder verzweigtes Alkyl mit bis zu 4 Kohlenstoffatomen steht, oder
- für Aralkyl mit 7 bis 14 Kohlenstoffatomen steht, das im Aromaten ein- oder mehrfach gleich oder verschieden durch Halogen oder Alkoxy mit bis zu 4 Kohlenstoffatomen substituiert sein kann, oder
- für eine Gruppe der Formel

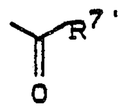 steht,

wobei
$R^{7'}$ - die oben angegebene Bedeutung hat,
oder
$R^5$, $R^6$ - gemeinsam eine Methylen-, Ethylen-, Propyliden-, Isopropyliden-, Cyclohexyliden- oder Benzylidengruppe bilden
und deren physiologisch unbedenkliche Salze.

7. Verwendung von 6-Hydroxymethylenverzeigten 3-Amino-4,5-dihydroxypiperidinen der allgemeinen Formel (I) gemäß Anspruch 1 zur Herstellung von Arzneimitteln.

8. Verbindungen der Formeln